(19) Europäisches Patentamt
European Patent Office
Office européen des brevets

(11) Publication number: **0 519 554 A1**

(12) **EUROPEAN PATENT APPLICATION**

(21) Application number: **92201693.6**

(22) Date of filing: **11.06.92**

(51) Int. Cl.5: **C07K 17/06**, C07K 3/28, A61K 39/385, A61K 39/21

(30) Priority: **19.06.91 US 715273**

(43) Date of publication of application: **23.12.92 Bulletin 92/52**

(84) Designated Contracting States: **CH DE FR GB IT LI NL**

(71) Applicant: **MERCK & CO. INC.**
**126, East Lincoln Avenue P.O. Box 2000**
**Rahway New Jersey 07065-0900(US)**

(72) Inventor: **Emini, A.**
**6 Faggs Manor Lane**
**Paoli, PA 19301(US)**
Inventor: **Liu, Margaret A.**
**4 Cushman Road**
**Rosemont, PA 19190(US)**
Inventor: **Marburg, Stephen**
**50 Concord Avenue**
**Metuchen, NJ 08840(US)**
Inventor: **Tolman, Richard L.**
**29 Upper Warren Way**
**Warren, NJ 07059(US)**

(74) Representative: **Thompson, John Dr. et al**
**Merck & Co., Inc. European Patent Department Terlings Park Eastwick Road**
**Harlow, Essex CM20 2OR(GB)**

(54) **Conjugates of the class II protein of the outer membrane of neisseria meningitidis and of HIV-1 related peptides.**

(57) The Class II major immuno-enhancing protein (MIEP) of Neisseria meningitidis, purified directly from the outer membrane of Neisseria meningitidis, or obtained through recombinant cloning and expression of DNA encoding the MIEP of Neisseria meningitidis, has immunologic carrier as well as immunologic enhancement and mitogenic properties. Conjugates of this protein and HIV-1 related peptides are useful for the induction of mammalian immune responses directed against the peptides, against HIV-1 strains, and for the neutralization of HIV-1 and prevention of HIV-I related diseases.

EP 0 519 554 A1

## BACKGROUND OF THE INVENTION

The outer membrane protein complex (OMPC) of Neisseria meningitidis is used as an immunologic carrier in vaccines for human use. OMPC consists of vesicles containing a variety of proteins as well as membranous lipids, including lipopolysaccharide (LPS or endotoxin).

OMPC has the property of immune enhancement, and when an antigen is chemically coupled to it, an increased antibody response to the antigen results. OMPC is currently used in vaccines for human infants against infectious agents such as Haemophilus influenzae as disclosed in Patent 4,695,624, and renders the infants capable of mounting an IgG and memory immune response to polyribosyl ribitol phosphate (PRP) of H. influenzae, when PRP is chemically coupled to OMPC.

OMPC is a mixture of a variety of proteins and lipids, and it was not known which component or components of OMPC bestows the beneficial immune enhancing effect to the coupled antigens. However, some potentially negative aspects of using OMPC in human vaccines include LPS related reactions such as fever, endotoxic shock, hypotension, neutropenia, activation of the alternative complement pathway, intravascular coagulation, and possibly death.

Furthermore, OMPC-antigen conjugates are quite heterogeneous in that the antigen may become conjugated to any of the protein moieties which make up OMPC, and the total protein per dose of a multivalent vaccine would be very high.

In U.S. application USSN 362,179, corresponding to EP-A-0402088; and 555,558; 555,974; 555,966; and 555,339, corresponding to EP-A-0467700 and EP-A-0467701, it was shown that HIV peptide-OMPC conjugates, or HIV peptide-polysaccharide-OMPC conjugates, or HIV peptide-OMPC-anion conjugates are useful for the induction of anti-peptide, or HIV-neutralizing immune responses. It has been discovered, as disclosed in copending application USSN 555,329, corresponding to EP-A-0467714, that MIEP, when chemically coupled to bacterial polysaccharide, functions as well as OMPC in inducing an antibody response to the polysaccharide. It was also discovered that MIEP is the Class II protein of the outer membrane of N. meningitidis. The Class II protein of N. meningitidis is a porin protein [Murakami, K., et al., (1989), Infection And Immunity, 57, pp.2318-23]. Porins are found in the outer membrane of all Gram negative bacteria.

The present invention extends the aforementioned discoveries by providing conjugates of MIEP and HIV related peptides.

## OBJECTS OF THE INVENTION

An object of the present invention is to provide a vaccine containing either the recombinant MIEP, or MIEP purified directly from the outer membrane of Neisseria meningitidis conjugated to peptides related to HIV-1. Another object of this invention is to provide conjugates that are highly immunogenic and are capable of raising an immune response in mammals specific to the epitopes presented by the peptidyl portion of the conjugates. Another object is to provide a covalent conjugate immunogen wherein the peptide portions of the peptide-protein conjugates are capable of eliciting mammalian immune responses which recognize HIV Principal Neutralizing Determinants. Another object is to provide a conjugate immunogen capable of raising mammalian antibody responses which neutralize the Human Immunodeficiency Virus. Another object is to provide a process for the high-yield production of covalent peptide-protein conjugates. Another object is to provide a method of using such conjugate immunogens to raise anti-peptide, anti-HIV, or HIV-neutralizing immune responses in mammalian recipients. Another object is to use vaccine formulations containing the conjugate of this invention to immunize humans prior to or after contraction of HIV infection or disease including AIDS. This and other objects will be apparent from the following description.

## SUMMARY OF THE INVENTION

This invention is a conjugate immunogen comprising the class II protein of the outer membrane of Neisseria meningitidis serogroup B, either derived by purification from the outer membrane protein complex of that organism, or from a recombinant host cell producing recombinant MIEP, and a peptide related to HIV-1, through a bigeneric, covalent spacer.

In one embodiment of the invention, the conjugates of the invention have the general structure:

$_j$(PEP-A-)-MIEP

or pharmaceutically acceptable salts thereof, wherein:

| | |
|---|---|
| PEP | is an HIV PND peptide, or a peptide capable of raising mammalian immune responses which recognize HIV PNDs; |
| MIEP | is the class II immunogenic protien of the outer membrane protein complex (OMPC) of Neisseria meningitidis b, either recombinantly produced or purified from the OMPC; |
| -A- | is a covalent linkage, preferably a bigeneric spacer; |
| j | is the peptide loading, and is the percentage by mass of peptide in the conjugate, and is between 1% and 90%, and preferably between 1% and 50% of the total protein mass in the conjugate. |

The conjugate of the invention is prepared by a process that utilizes the available nucleophilic functionalities, found in proteins, such as the amino group of lysine, the imidazole group of histidine, or the hydroxyl groups of serine, threonine, or tyrosine. The process can be carried out in several ways in which the sequence, method of activation, and reaction of protein and peptide groups can be varied. In a preferred embodiment the process comprises the steps of:

Process 1:

1a. reacting the protein nucleophilic groups with a reagent, for example with N-acetyl homocysteine thiolactone, which generates thiol groups on the protein; and
1b. reacting the product of step 1a. with peptides previously derivatized so as to append an electrophilic group, preferably comprising maleimide, on the peptide; or

Process 2:

2a. reacting the protein nucleophilic groups with a bifunctional electrophilic reagent, such as maleimidoalkanoic acid hydroxysuccinimide ester, so as to generate an electrophilic protein; and
2b. reacting the product of step 2a with a peptide containing a nucleophile, such as a thiol group.

DEFINITIONS AND ABBREVIATIONS

| | |
|---|---|
| AA assay | amino acid analysis method wherein peptides or proteins are acid hydrolyzed to the free amino acids and then quantitated |
| Ac | Acetyl |
| Acm | acetamidomethyl thiol protecting group |
| activation | reaction of peptides, proteins, or anionic moieties with a reagent capable of derivatizing the moiety in order to enable subsequent desirable reactions to occur |
| AIDS | Acquired Immune Deficiency Syndrome |
| amino acid | a molecule having both an acid and amino functional group; there are 20 common $\alpha$-amino acids with the general structure $H_2N$-CHR-COOH, wherein the R group defines the identity of the amino acid; these amino acids may have either a D or L stereochemical form and unless specified otherwise, by the lower-case one letter abbreviation, or by the prefix "D-" before an amino acid name, the amino acid is of the natural or L configuration; the names of the 20 common amino acids and the structure of the R group are identified herein in single-letter code according to the following table: |

| AMINO ACID NAME | 3-letter code | 1-letter code | side-chain (R) |
|---|---|---|---|
| Alanine | Ala | A | $-CH_3$ |
| Arginine | Arg | R | $-(CH_2)_3NHCHNH_2NH_2^+$ |
| Asparagine | Asn | N | $-CH_2CONH_2$ |
| Aspartic Acid | Asp | D | $-CH_2COOH$ |
| Cysteine | Cys | C | $-CH_2SH$ |
| Glutamic Acid | Glu | E | $-(CH_2)_2COOH$ |
| Glutamine | Gln | Q | $-(CH_2)_2CONH_2$ |
| Glycine | Gly | G | -H |
| Histidine | His | H | $-CH_2$-imidazole |
| Isoleucine | Ile | I | $-CH(CH_3)CH_2CH_3$ |
| Leucine | Leu | L | $-CH_2CH(CH_3)_2$ |
| Lysine | Lys | K | $-(CH_2)_4NH_3^+$ |
| Methionine | Met | M | $-(CH_2)_2SCH_3$ |
| Phenylalanine | Phe | F | $-CH_2$-Phenyl |
| Proline | Pro | P | -$\alpha$,N-trimethylene |
| Serine | Ser | S | $-CH_2OH$ |
| Threonine | Thr | T | $-CH(OH)CH_3$ |
| Tryptophan | Trp | W | $-CH_2$-indole |
| Tyrosine | Tyr | Y | $-CH_2$-phenyl-OH |
| Valine | Val | V | $-CH(CH_3)_2$ |
| antibody | a protein produced by mamalian B cells that is capable of binding a particular antigen | | |

| | |
|---|---|
| ARC | AIDS-Related Complex |
| AZT | Azidothymidine, an anti-AIDS compound |
| bigeneric spacer | a molecular chain resulting from the reaction of separately derivatized partners; analytical degradation of the coconjugate formed through the spacer allows release and quantitation of the spacer, providing a measure of the degree of covalent attachment |
| BOP | Benzotriazol-1-yloxytris(dimethylamino)phosphonium hexafluorophosphate |
| capping | the elimination of reactive sites on a coconjugate by reaction with small molecules |
| Cbz | benzyloxycarbonyl |
| conjugate | a complex of discrete chemical entities covalently bound one to the other, wherein at least one entity is a desired antigen (e.g. an HIV PND) and another entity is a carrier |
| core amino acids | those amino acids of an HIV PND which are essential for inducing HIV-neutralizing immune responses in a mammal, and comprise a part of the loop amino acids |
| DPPA | diphenylphosphorylazide |
| ELISA | enzyme-linked immunoadsorbant assay |
| fmoc | 9-fluorenylmethoxycarbonyl |
| HIV | Human Immunodeficiency Virus, a member of the lentivirus group and the purported etiologic agent implicated in AIDS and related complexes; HIV is alternatively known as HTLV (Human T-cell Lymphocyto-trophic Virus) III, LAV (Lymphadenopathy Associated Virus), and ARV (AIDS Related Virus) |
| HIV Disease | Clinically recognized disease state characterized by the presence or any of a number of phyorologic dysfunctions known to be associated with HIV infections. |
| immunogen | a molecule useful as a stimulator of a mammalian immune response |

| | |
|---|---|
| immunologically equivalent peptides | cyclic or linear peptides having in common the function of eliciting HIV neutralizing immune responses in mammals, such as antibodies, which are able to recognize HIV PND epitopes |
| loop amino acids | amino acids comprising the core amino acids in a cyclic HIV PND peptide which go toward formation of the peprtide cycle; |
| marker amino acid | an amino acid having a signal in the AA assay which is free of interference by signals generated by other peptide or protein amino acids, for example, norleucine, b-alanine, gamma amino butyric acid, 6-aminohexanoic acid, ornithine |
| MIEP | Major Immuno Enhancing Protein |
| Mtr | 4-methoxy-2,3,6-trimethyl phenyl sulfonyl |
| NEM | N-ethylmaleimide |
| OMPC | Outer Membrane Protein Complex of Neisseria meningitidis; used as an immunoenhancer and peptide carrier |
| peptide | a polymer of amino acids linked by amide (peptide) bonds |
| PEP | peptide |
| PND | Principal Neutralizing Determinant; the name attributed to peptidyl sequences capable of binding to HIV neutralizing antibodies and capable of raising HIV-neutralizing antibodies in a mammalian recipient upon inoculation with an immunogen containing the PND |
| PRO | an immunogenic protein |
| protein | a large peptide |
| resins | solid support matrices for solid phase peptide synthesis<br>Wang:<br>4-(hydroxymethyl)phenoxymethyl linkage to copolystyrene-1%divinylbenzene resin, which is used for batch Fmoc solid phase peptide synthesis, with final 95% TFA cleavage from the resin and concomitant deprotection of acid sensitive side chain protecting groups;<br>Sasrin:<br>4-(hydroxymethyl)-3-methoxyphenoxymethyl linkage to copolystyrene-1%divinylbenzene resin, which is used for batch Fmoc solid phase peptide synthesis, with final 1% TFA/$CH_2Cl_2$ cleavage from the resin, leaving intact acid labile side chain protecting groups;<br>Pepsyn KA:<br>4-(hydroxymethyl)phenoxymethyl linkage to polyamide resin adsorbed on to kieselguhr, which is used for continuous flow column Fmoc solid phase peptide synthesis. Peptides are cleaved from the resin as described above for Wang resin;<br>Pepsyn KH:<br>4-(hydroxymethyl)-3-methoxymethyl linkage to polyamide resin adsorbed on to kieselguhr, which is used for Fmoc solid phase peptide synthesis. Side chain protected peptides are cleaved from the resin as described above for the Sasrin resin |
| SCMHC | S-carboxymethyl homocysteamine, an acid-stable bigeneric spacer released by degradation of covalent coconjugate immunogens and quantifiable by AA assay |
| SCMC | S-carboxymethyl cysteamine, an acid-stable bigeneric spacer released by degradation of covalent coconjugate immunogens and quantifiable by AA assay |
| Z | benzyloxycarbonyl |

## DETAILED DESCRIPTION OF THE INVENTION

It is known that certain substances which by themselves elicit an immune response which consists of only IgM class antibodies and no memory, can be transformed into fully immunogenic antigens which elicit

IgM and IgG anitbodies as well as memory, by chemical coupling to a strongly (T-cell dependent) antigenic substance. This immunologic phenomenon is termed the "carrier effect", while the weak or non-immunogenic moiety, and the strongly antigenic substance are termed "hapten" and "carrier", respectively.

Injection of the hapten-carrier complex into an animal will result in the formation of antibodies by B-lymphocytes, some of which will be specific for, and bind to the hapten, and others which will be specific for, and bind to the carrier. An additional aspect of the carrier effect is that upon a subsequent exposure to the hapten-carrier complex, a vigourous antibody response to the hapten ensues. This is termed a memory, or anamnestic response.

The carrier effect appears to involve functions mediated by certain T-lymphocytes, called "helper T-lymphocytes". The carrier molecule stimulates the helper T-lymphocytes to assist, in some way, formation of anti-hapten IgG class antibody-producing B-lymphocytes and a memory response.

Helper T-lymphocytes are normally involved in the production by B-lymphocytes, of antibodies specific for a certain type of antigens, termed "T-dependent" antigens, but not for other antigens termed "T-independent" antigens. A carrier molecule can convert a T-independent, weak or non-immunogenic hapten into a T-dependent, strongly antigenic molecule. Furthermore, a memory response will follow a subsequent exposure to the hapten-carrier complex and will consist primarily of IgG, which is characteristic of T-dependent antigens and not T-independent antigens.

The utility of carrier molecules is not limited to use with T-independent antigens but can also be used with T-dependent antigens. The antibody response to a T-dependent antigen may be enhanced by coupling the antigen to a carrier, even if the antigen can, by itself, elicit an antibody response.

Certain other molecules have the ability to generally stimulate the overall immune system. These molecules are termed "mitogens" and include plant proteins as well as bacterial products. Mitogens cause T and/or B-lymphocytes to proliferate, and can broadly enhance many aspects of the immune response including increased phagocytosis, increased resistance to infection, augmented tumor-immunity, and increased antibody production.

Many infectious disease causing agents can, by themselves, elicit protective antibodies which can bind to and kill, render harmless, or cause to be killed or rendered harmless, the disease causing agent and its byproducts. Recuperation from these diseases usually results in long lasting immunity by virtue of protective antibodies generated against the highly antigenic components of the infectious agent.

Protective antibodies are part of the natural defense mechanism of humans and many other animals, and are found in the blood as well as in other tissues and bodily fluids. It is the primary function of most vaccines to elicit protective antibodies against infectious agents and/or their byproducts, without causing disease.

OMPC from N. meningitidis has been used successfully to induce antibody responses in humans when OMPC is chemically coupled to T-cell independent antigens, including bacterial polysaccharides. OMPC contains several bacterial outer membrane proteins as well as bacterial lipids. In addition, OMPC has a vesicular three dimensional structure.

The efficacy of OMPC as an immunologic carrier was thought to depend on one or more of the bacterial membrane proteins, bacterial lipids, the liposomal three dimensional structure, or a combination of bacterial proteins, lipids, and vesicular structure. The instant invention utilizes one of the OMPC proteins, MIEP, which possesses the immunologic carrier and immune enhancement properties of OMPC and is effective in purified form, free from other N. meningitidis membrane proteins and lipopolysaccharides.

The conjugate of this invention may be supplied in an aqueous phase which may conveniently be comprised of the antigenic material in a parenterally acceptable liquid. For example, the aqueous phase may be in the form of a vaccine in which the antigen is dissolved in a balanced salt solution, physiological saline solution, phosphate buffered saline solution, tissue culture fluids, or other media in which an organism may have been grown. The aqueous phase also may contain preservatives and/or substances conventionally incorporated in vaccine preparations. Adjuvant emulsions containing MIEP conjugated antigen may be prepared employing techniques well known in the art.

MIEP can be purified from OMPC derived from cultures of N. meningitidis grown in the usual manner as described in U.S. Patent number 4,459,286 and U.S. Patent number 4,830,852. OMPC purification can be done according to the methods described in U.S. Patent number 4,271,147, 4,459,286, and 4,830,852.

MIEP can also be obtained from recombinant DNA engineered host cells by expression of recombinant DNA encoding MIEP. The DNA encoding MIEP can be obtained from N. meningitidis cells [Murakami, K. et al., (1989), Infection And Immunity, 57, pp. 2318], or the DNA can be produced synthetically using standard DNA synthesis techniques. DNA encoding MIEP can be expressed in recombinant host cells including but not limited to bacteria, yeast, insect, mammalian or other animal cells, yielding recombinant MIEP. The preferred methods of the present invention for obtaining MIEP are purification of MIEP from OMPC and

recombinant DNA expression of DNA encoding MIEP derived from N. meningitidis, with purification from OMPC most preferred.

Purified MIEP was prepared from OMPC vesicles by sodium dodecylsulfate (SDS) lysis of the vesicles followed by SDS polyacrylamide gel electrophoresis (PAGE). The MIEP was eluted from the gel, dialysed against a high pH buffer and concentrated. Standard methods of polyacrylamide gel electrophoresis can be utilized to purify MIEP from OMPC vesicles. Such methods are described in Molecular Cloning: A Laboratory Manual, Sambrook, J. et al., (1989), Cold Spring Harbor Laboratory Press, New York, and Current Protocols In Molecular Biology, (1987) Ausubel F.M. et al., editors, Wiley and Sons, New York.

Standard methods of eluting proteins from SDS-polyacrylamide gels are described in Hunkapiller, M.W., and Lujan, E., (1986), Purification Of Microgram Quantities Of Proteins By Polyacrylamide Gel Electrophoresis, in Methods of Protein Microcharacterization (J. Shively editor) Humanna Press, Clifton N.J., and Current Protocols In Molecular Biology (1987), Ausubel, F.M., et al., editors, Wiley and Sons, New York.

MIEP prepared in this manner is readily suitable for conjugation to antigens derived from bacteria, viruses, mammalian cells, rickettsia, allergens, poisons or venoms, fungi, peptides, proteins, polysaccharides, or any other antigen.

Recombinant MIEP can be prepared by expression of genomic N. meningitidis DNA encoding MIEP in bacteria, for example E. coli or in yeast, for example S. cerevisiae. To obtain genomic DNA encoding MIEP, genomic DNA is extracted from N. meningitidis and prepared for cloning by either random fragmentation of high molecular weight DNA following the technique of Maniatis, T. et al., (1978), Cell, 15, pp. 687, or by cleavage with a restriction endonuclease by the method of Smithies, et al., (1978), Science, 202, pp. 1248. The genomic DNA is then incorporated into an appropriate cloning vector, for example lambda phage [see Sambrook, J. et al., (1989), Molecular Cloning, A Laboratory Manual. Cold Spring Harbor Press, New York]. Alternatively, the polymerase chain reaction (PCR) technique (Perkin Elmer) can be used to amplify specific DNA sequences in the genomic DNA [Roux, et al., (1989), Biotechniques, 8, pp. 48]. PCR treatment requires a DNA oligonucleotide which can hybridize with specific DNA sequences in the genomic DNA. The DNA sequence of the DNA oligonucleotides which can hybridize to MIEP DNA in the N. meningitidis genomic DNA can be determined from the amino acid sequence of MIEP or by reference to the determined DNA sequence for the Class II major membrane protein of N. meningitidis [Musakami, k. et al., (1989), Infection and Immunity, 57, pp. 2318].

Recombinant MIEP can be separated from other cellular proteins by use of an affinity column made with monoclonal or polyclonal antibodies specific for MIEP. These affinity columns are made by adding the antibodies to Affigel-10 (Biorad), a gel support which is pre-activated with N-hydroxysuccinimide esters such that the antibodies form covalent linkages with the agarose gel bead support. The antibodies are then coupled to the gel via amide bonds with the spacer arm. The remaining activated esters are then quenched with 1M ethanolamine HCl (pH 8). The column is washed with water followed by 0.23 M glycine HCl (pH 2.6) to remove any non-conjugated antibody or extraneous protein. The column is then equilibrated in phosphate buffered saline (pH 7.3) and the cell culture supernatants or cell extracts containing MIEP are slowly passed through the column. The column is then washed with phosphate buffered saline until the optical density ($A_{280}$) falls to background, then the protein is eluted with 0.23 M glycine-HCl (pH 2.6). The protein is then dialyzed against phosphate buffered saline.

In one embodiment of the invention, the novel conjugate of this invention comprises MIEP, the major immuno enhancing protien of the outer membrane protein complex (OMPC) of Neisseria meningitidis b, covalently linked to HIV PND peptides.

In one embodiment, the conjugates are prepared by the process of covalently coupling activated peptide to an activated protein. The peptide and protein components are separately activated to display either pendant electrophilic or nucleophilic groups so that covalent bonds will form between the peptide and the protein upon contact.

The covalent conjugate immunogens that result from the series of reactions described above may conveniently be thought of as a conjugate in which multiple peptide functionalities are built upon a foundation of MIEP. In other embodiments of the invention, the peptide may be linked to the MIEP through the intermediary of an anionic polysaccharide, such as polyribosyl ribitol phosphate. In yet another embodiment, anionic functionalities may be appended to MIEP to provide anionic character to the conjugate. Such anionic character is thought to be useful in counteracting cationic character of conjugated peptides.

When the peptide components of the conjugate are capable of eliciting HIV neutralizing immune responses, the conjugates of this invention may be administred to mammals in immunologically effective amounts, with or without additional immunomodulatory, antiviral, or antibacterial compounds, and are useful for inducing mammalian immune responses against the peptidyl portion of the conjugates, for inducing HIV-neutralizing antibodies in mammals, or for making vaccines for administration to humans to prevent contraction of HIV infection or disease including AIDS, or for administration to humans afflicted with HIV infection or disease including AIDS.

In a preferred embodiment, the conjugate of the invention has the general structure:

$_j$(PEP-A-)-MIEP

or pharmaceutically acceptable salts thereof, wherein:

- PEP is an HIV PND peptide, or a peptide capable of raising mammalian immune responses which recognize HIV PNDs;
- MIEP is an immunogenic protein of the outer membrane protein complex (OMPC) of Neisseria meningitidis b either recombinatly produced or purfied from OMPC;
- -A- is a covalent linkage, preferably a bigeneric spacer;
- j is the percentage by mass of peptide in the coconjugate, and is preferably between 1% and 50% of the total protein mass in the conjugate.

The conjugate of the invention may be prepared by any of the common methods known in the art for preparation of peptide-protien conjugates, such as, for example, the bigeneric chemistry disclosed in U.S. patent 4,695,624 and Marburg et al. J.A.C.S. 108, 5282 (1986), and in Applications USSN 362,179; 555,558; 555,974; 555,966 and 555,339, corresponding to EP-A-0402088, EP-A-0467700 and EP-A-0467701. In a preferred embodiment, a process that utilizes the available nucleophilic functionalities, found in proteins, such as the amino group of lysine, the imidazole group of histidine, or the hydroxyl groups of serine, threonine, or tyrosine is used. In practical terms, the number of available protien necleophilic sites may be determined by an appropriate assay which may comprise thiolation with N-acetyl homocysteine thiolactone, followed by Ellman Assay [Ellman, G.L., Arch. Biochem. Biophys., 82, 70 (1959)] for determination of total free sulfydryl groups and/or by alkylation with a bromoacetyl amino acid, assayable by amino acid analysis.

The preferred process can be carried out in several ways in which the sequence, method of activation, and reaction of protein and peptide groups can be varied. The process may comprise the steps of:

Process 1:

1a. reacting the protein nucleophilic groups with a reagent, for example with N-acetyl homocysteine thiolactone, which generates thiol groups on the protein; and

1b. reacting the product of step 1a. with peptides previously derivatized so as to append an electrophilic group preferably comprising moleimide, on the peptide. A preferred embodiment of this invention, which may be prepared according to this process, has the structure:

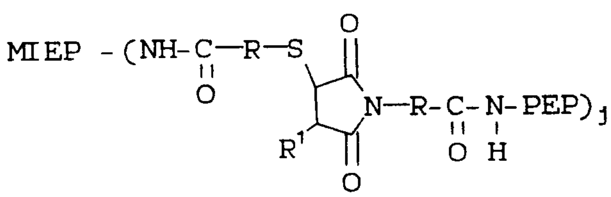

or pharmaceutically acceptable salts thereof, wherein:
PEP, MIEP, and j, are as defined supra;

-R- is:
- a) -lower alkylene-,
- b) -substituted lower alkylene-,
- c) -cycloalkylene-,
- d) -substituted cyloalkylene-,
- e) -phenylene-;

-$R^1$ is:
- a) -hydrogen,
- b) -lower alkyl, or
- c) -$SO_3H$; and

-S- is sulfur.

Likewise, a preferred embodiment of the invention having the structure:

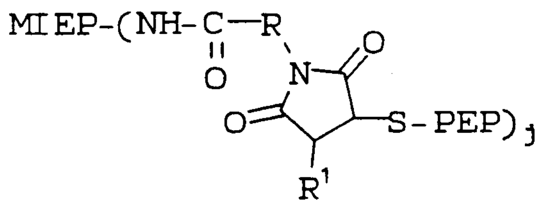

wherein all variables are as defined above, may be prepared by process 2, which comprises the steps of:

2a. reacting the protein nucleophilic groups with a bifunctional electrophilic reagent, such as maleimidoalkanoic acid hydroxysuccinimide ester, so as to generate an electrophilic protein; and

2b. reacting the product of step 2a. with a peptide containing a nucleophile, such as a thiol group.

A highly preferred embodiment of process 1, is described in detail below and in Scheme A. According to the scheme, the immunogenic protein is the class II protein of the outer membrane protein complex (OMPC) of Neisseria meningitidis b, either purified from the bacterial membrane or produced by recombinant means. The process comprises the steps of:

a.i. reacting MIEP (I), having nucleophilic groups, including free amino groups due to the presence of lysines or protein amino-termini, with a thiolating agent, preferably N-acetyl homocysteine thiolactone, to generate MIEP (II) having "m" moles of sulfhydryl groups available for reaction with a thiophile; a.ii. quantitating the number of available sulfhydryls appended to MIEP in step 1a.i. to determine the value of "m", preferably by Ellman assay [Ellman, G.L., Arch. Biochem. Biochem. Biophys., 82, 70 (1959)]; and

b. contacting the product of step a. with an excess, (≧m), of an HIV PND which has been previously derivatized so as to append an electrophilic group, preferably with a maleimido-alkanoic acid, and most preferably with maleimido-propionic acid (this derivatization is achieved by N-protecting all amino groups on the peptide that should not be derivatized, and reacting the free peptide amino groups with a bifunctional reagent, preferably maleimidoalkanoyloxysuccinimide, and most preferably maleimidopropionyloxysuccinimide), to generate the conjugate of this invention (III).

The conjugate product may be purified by, for example, dialysis in a buffer having an ionic strength between 0.001M and 1M and a pH between 4 and 11, and most preferably in an aqueous medium having an ionic strength of between 0.01 and 0.1M and a pH of between about 6 and 10.

SCHEME A

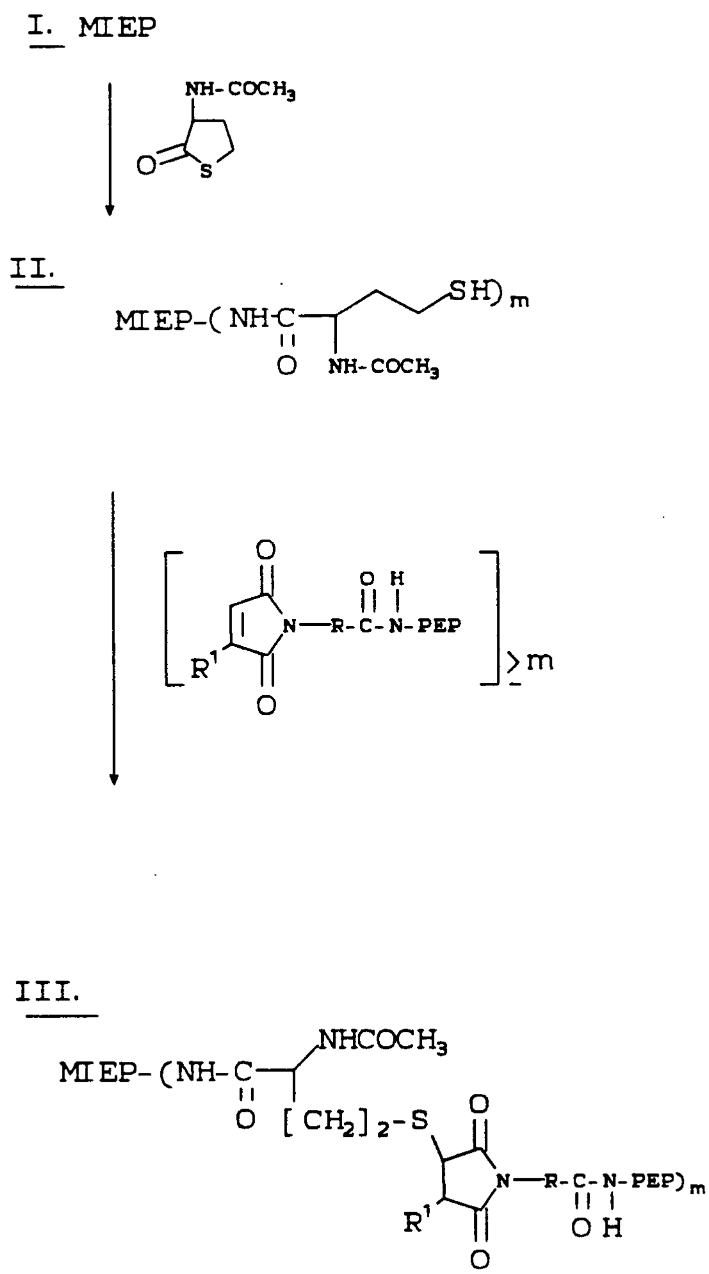

The process described above and depicted in Scheme A may be modified so that MIEP is derivatized so as to be covalently linked to a thiophile, such as a derivative of maleimide, while the peptide is activated so as to be covalently linked to free sulfhydryls. This and other alternate processes, naturally fall within the scope of this disclosure, including variations on these processes, such as variations of sequence of reaction of activated species, or ratios of reactants.

The process for making the conjugates of this invention may be applied to making any conjugate wherein a peptide-protein conjugate is desired and is particularly significant where enhanced immunogenicity of the peptide is required.

The conjugates herein described may be included in compositions containing an inert carrier and are useful when appropriately formulated as a vaccine. This may include prior adsorption onto alum or combination with emulsifiers or adjuvants known in the art of vaccine formulation. Methods of using the covalent conjugate immunogens of this invention include: (a) use as a laboratory tool to characterize HIV PND peptide structure-function relationships; (b) use as an immunogen to raise HIV-neutralizing antibodies in a mammal which antibodies may be isolated and administered to a human so as to prevent infection by HIV, or to limit HIV proliferation post-infection, or to treat humans afflicted by HIV infection or disease including AIDS. (c) use as a vaccine to immunize humans against infection by HIV or to treat humans post-infection, or to boost an HIV-neutralizing immune response in a human afflicted with HIV infection or disease including AIDs.

As a laboratory tool, the conjugate is useful when administered to a mammal in an immunologically effective amount, to generate anti-PND peptide, anti-HIV, or HIV-neutralizing immune responses. The mammal may be boosted with additional conjugate to elevate the immune response. Antiserum is obtained from such a mammal by bleeding the mammal, centrifuging the blood to separate the cellular component from the serum, and isolating antibody proteins from the serum if necessary, according to methods known in the art. Such antiserum or antibody preparations may be used to characterize the efficacy of an HIV PND peptide in a conjugate in raising mammalian anti-PND peptide, anti-HIV, or HIV-neutralizing antibodies in a mammal. ELISA assays using the unconjugated peptide and the antiserum are useful in vitro assays for measuring the elicitation of anti-peptide antibodies. An in vitro assay for measuring the HIV-neutralizing ability of antiserum comprises incubating a preparation of live HIV with a preparation of the antiserum, then incubating the antiserum-treated HIV preparation with CD4 receptor bearing cells, and measuring the extent of cellular protection afforded by the antiserum. These assays and the characteristics of antiserum produced by a given conjugate may be used to study the PND peptide stucture-function relationship.

The conjugate is useful for inducing mammalian antibody responses as described in the previous paragraph, and such antibodies may be used to passively immunize humans to prevent HIV infection, or to limit HIV proliferation post-infection, or to treat humans afflicted with HIV infection or disease including AIDS.

The conjugate is useful as a vaccine which may be administered to humans to prevent HIV infection or proliferation, or to humans suffering from HIV disease of HIV infection, including AIDS and related complexes, or to humans testing seropositive for the HIV virus. The conjugate may be administered in conjunction with other anti-HIV compounds, such as AZT, or more general anti-viral compounds, or in conjunction with other vaccines, antibiotics, or immunomodulators (see Table I below).

The form of the immunogen within the vaccine takes various molecular configurations. A single molecular species of the antigenic conjugate III will often suffice as a useful and suitable antigen for the prevention or treatment of HIV disease including AIDS or ARC. Other antigens in the form of cocktails are also advantageous, and consist of a mixture of conjugates that differ by, for example, the mass ratio of peptide to total protein. In addition, the conjugates in a mixture may differ in the amino acid sequence of the PND.

An immunological vector, carrier or adjuvant may be added as an immunological vehicle according to conventional immunological testing or practice.

Adjuvants may or may not be added during the preparation of the vaccines of this invention. Alum is the typical and preferred adjuvant in human vaccines, especially in the form of a thixotropic, viscous, and homogeneous aluminum hydroxide gel. For example, one embodiment of the present invention is the prophylactic vaccination of patients with a suspension of alum adjuvant as vehicle and a cocktail of conjugates as the selected set of immunogens or antigens.

The vaccines of this invention may be effectively administered, whether at periods of pre-exposure or post-exposure, in combination with effective amounts of the AIDS antivirals, immunomodulators, antibiotics, or vaccines of Table I [source: Market Letter, Nov. 30, 1987, p. 26-27; Genetic Engineering News, Jan. 1988, Vol. 8, p. 23.]

## TABLE I[1]

### A. Antivirals

| Drug Name | Manufacturer | Indication |
|---|---|---|
| AL-721 | Ethigen | ARC, PGL |
| BETASERON (interferon beta) | Triton Biosciences | AIDS, ARC, KS |
| CARRISYN (polymannoacetate) | Carrington Labs | ARC |
| CYTOVENE (ganciclovir) | Syntex | CMV |
| DDC (dideoxycytidine) | Hoffmann-La Roche | AIDS, ARC |
| FOSCARNET (trisodium phosphonoformate) | Astra AB | HIV inf, CMV retinitis |
| HPA-23 | Rhone-Poulenc Sante | HIV infection |

---

[1]Abbreviations: AIDS (Acquired Immune Deficiency Syndrome); ARC (AIDS related complex); CMV (Cytomegalovirus, which causes an opportunistic infection resulting in blindness or death in AIDS patients); HIV (Human Immunodeficiency Virus, previously known as LAV, HTLV-III or ARV); KS (Kaposi's sarcoma); PCP (Pneumonocystis carinii pneumonia, an opportunistic infection); PGL (persistent generalized lymphadenopathy).

| Drug Name | Manufacturer | Indication |
|---|---|---|
| ORNIDYL (eflornithine) | Merrell Dow | PCP |
| PEPTIDE T (octapeptide sequence) | Peninsula Labs | AIDS |
| RETICULOSE (nucleophosphoprotein) | Advanced Viral Research | AIDS, ARC |
| IR (zidovudine; AZT) | Burroughs Wellcome | AIDS, advanced ARC pediatric AIDS, KS, asympt HIV, less severe HIV, neurological involvement. |

| | | |
|---|---|---|
| RIFABUTIN (ansamycin LM 427) | Adria Labs | ARC |
| (trimetrexate) | Warner-Lambert | PCP |
| UA001 | Ueno Fine Chem Industry | AIDS, ARC |
| VIRAZOLE (ribavirin) | Viratek/ICN | AIDS, ARC, KS |
| WELLFERON (alfa interferon) | Burroughs Wellcome | KS, HIV, in comb with RETROVIR |
| ZOVIRAX (acyclovir) | Burroughs Wellcome | AIDS, ARC, in comb with RETROVIR |

B. Immunomodulators

| Drug Name | Manufacturer | Indication |
|---|---|---|
| ABPP (bropirimine) | Upjohn | Advanced AIDS, KS |
| AMPLIGEN (mismatched RNA) | DuPont HEM Research | ARC, PGL |
| (Anti-human alpha interferon antibody) | Advanced Biotherapy Concepts | AIDS, ARC, KS |

| | | |
|---|---|---|
| Colony Stimulating Factor (GM-CSF) | Sandoz Genetics Institute | AIDS, ARC, HIV, KS |
| CL246,738 (CL246,738) | American Cynamid | AIDS |
| IMREG-1 | Imreg | AIDS, ARC, PGL, KS |
| IMREG-2 | Imreg | AIDS, ARC, PGL, KS |
| IMUTHIOL (diethyl dithio carbamate) | Merieux Institute | AIDS, ARC |
| IL-2 (interleukin-2) | Cetus | AIDS, KS |

| Drug Name | Manufacturer | Indication |
|---|---|---|
| IL-2 (interleukin-2) | Hoffmann-La Roche Immunex | AIDS, KS |
| INTRON-A (interferon alfa) | Schering-Plough | KS |
| ISOPRINOSINE (inosine pranobex) | Newport Pharmaceuticals | ARC, PGL, HIV seropositive patients |
| (methionine enkephalin) | TNI Pharmaceuticals | AIDS, ARC |

| | | |
|---|---|---|
| MTP-PE (muramyl-tripeptide) | Ciba-Geigy | KS |
| THYMOPENTIN (TP-5) (thymic compound) | Ortho Pharmaceuticals | HIV infection |
| ROFERON (interferon alfa) | Hoffmann-La Roche | KS |
| (recombinant erythropoietin) | Ortho Pharmaceuticals | severe anemia assoc with AIDS & RETROVIR therapy |
| TREXAN (naltrexone) | DuPont | AIDS, ARC |
| TNF (tumor necrosis factor) | Genentech | ARC, in combination interferon gamma |

C. Antibiotics

| | | |
|---|---|---|
| PENTAM 300 (pentamidine isethionate) | LyphoMed | PCP |

D. Vaccines

| | | |
|---|---|---|
| Gag | Merck | AIDS,ARC |

It will be understood that the scope of combinations of the vaccines of this invention with AIDS antivirals, immunomodulators, antibiotics or vaccines is not limited to the list in the above Table, but includes in principle any combination with any pharmaceutical composition useful for the treatment of AIDS. The AIDS or HIV vaccines of this invention include vaccines to be used pre- or post-exposure to prevent or treat HIV infection or disease, and are capable of producing an immune response specific for the immunogen.

The conjugates of this invention, when used as a vaccine, are to be administered in immunologically effective amounts. Dosages of between 1 $\mu$g and 500 $\mu$g of conjugate protein, and preferably between 50 $\mu$g and 300 $\mu$g of conjugate protein are to be administered to a mammal to induce anti-peptide, anti-HIV, or HIV-neutralizing immune responses. About two weeks after the initial administration, a booster dose may be administered, and then again whenever serum antibody titers diminish. The conjugate should be administered intramuscularly or by any other convenient or efficacious route, at a concentration of between 10

µg/ml and 1 mg/ml, and preferably between 50 and 500 µg/ml, in a volume sufficient to make up the total required for immunological efficacy. The conjugate may be preadsorbed to aluminum hydroxide gel or to the Ribi adjuvant (GB 2220211A, US priority document 212,919 filed 29/06/1988) and suspended in a sterile physiological saline solution prior to injection.

The protein moiety should behave as an immune enhancer. It is desirable, in the choice of protein, to avoid those that result in non-specific activation of the recipient's immune response (reactogenicity). In U.S. Patent 4,695,624, Marburg et al. used the outer membrane protein complex (OMPC) derived from Neisseria meningitidis to prepare polysaccharide-protein conjugates. OMPC has proven to be suitable though other immunogenic proteins may be used. The instant invention utilizes the Class II major immune enhancing protein (MIEP) of OMPC.

Various methods of purifying OMPC from the gram-negative bacteria have been devised [Frasch et al., J. Exp. Med. 140, 87 (1974); Frasch et al., J. Exp. Med. 147, 629 (1978); Zollinger et al., US Patent 4,707,543 (1987); Helting et al., Acta Path. Microbiol. Scand. Sect. C. 89, 69 (1981); Helting et al., US Patent 4,271,147]. OMPC may be used herein essentially according to the Helting process, from which MIEP may be further purified [Murakami, K., et al., Infection and Immunity, 57, 2318 (1989)], to provide immune enhancement necessary to induce mammalian immune responses to HIV PND peptides. MIEP may be derived by dissociation of the isolated OMPC, or alternatively, produced through recombinant expression of the desired immunogenic portions of OMPC. Methods of preparing and using an OMPC subunit are disclosed in co-pending US application serial Nos. 555,329; 555,978; and 555,204, corresponding to EP-A-0467714.

The HIV PND peptides that may be used for making species of the conjugate of this invention may be linear or cyclic peptides. The linear peptides may be prepared by known solid phase peptide synthetic chemistry, by recombinant expression of DNA encoding desireable peptide sequences, or by fragmentation of isolated HIV proteins. Cyclic HIV PND peptides may be prepared by cyclization of linear peptides, for example (a) by oxidizing peptides containing at least two cysteines to generate disulfide bonded cycles; (b) by forming an amide bonded cycle; (c) by forming a thioether bonded cycle. Processes for making such peptides are described herein but this description should not be construed as being exhaustive or limiting. The conjugates of this invention are useful whenever a component peptide is an HIV PND or is capable of priming mammalian immune responses which recognize HIV PNDs.

PND peptides, both those known in the art and novel compounds disclosed herein and separately claimed in co-pending U.S. Application Serial Nos. 555,112 and 555,227 and 715,127, corresponding to EP-A-0471453, EP-A-0467699 and EP-A-0467701, are defined as peptidyl sequences capable of inducing an HIV-neutralizing immune response in a mammal, including the production of HIV-neutralizing antibodies.

A major problem overcome by the instant invention is the HIV interisolate sequence variability. For example, in the PND which occurs in the third hypervariable region of gp120 (see below), although certain amino acids have been found to occur at given locations in a great many isolates, no strictly preserved primary sequence motif exists. This difficulty is surmounted by this invention because it allows conjugation of a cocktail of peptides having PND sequences from as many different HIV isolates as necessary to attain broad protection. Alternatively, a broadly protective cocktail of conjugates may be prepared by mixing conjugates, each of which is prepared separately with a peptide moiety providing protection against a single or several HIV isolates.

The amino acids found near or between amino acids 296 and 341 of gp120 have been shown to meet the criteria which define a PND. In the IIIB isolate of HIV, a 41-amino-acid sequence has been reported as follows (Seq ID No: 1:):

-Ile Asn Cys Thr Arg·Pro Asn Asn Asn Thr Arg Lys Ser
Ile Arg Ile Gln Arg Gly Pro Gly Arg Ala Phe Val Thr
Ile Gly Lys Ile Gly Asn Met Arg Gln Ala His Cys Asn
Ile Ser-,

with the two cysteines being disulfide bonded to each other to form a loop. The trimer -Gly Pro Gly- is exposed at the PND loop tip. Peptides from different HIV isolates from this same region of gp120 raise isolate-specific neutralizing antibodies when presented to goat and guinea pig immune systems as conjugates with keyhole-limpet hemocyanin. The major neutralizing epitope within the 41-mer sequence, presented above, is comprised by the eight amino acids surrounding and including the -Gly Pro Gly- trimer

[Javaherian et al., PNAS USA 86, 6768 (1989)]. In Table II below a number of linear peptides of different length and composition that can be used to prepare the conjugates of this invention are presented. The name of the isolate containing a peptide having the sequence of the tabulated peptide is given, along with a name herein ascribed to that peptide for ease of reference. The letter r- on the left hand side of each peptide represents the possibility of linking the peptide to an immunogenic protein, such as the MIEP at that position. In addition, marker amino acids, such as norleucine and ornithine may form part of r-.

TABLE II

LINEAR HIV PND PEPTIDES

| HIV Isolate | Peptide Sequence | Name | SEQ ID NO: |
|---|---|---|---|
| MN | r-Tyr Asn Lys Arg Lys Arg Ile His Ile Gly Pro Gly Arg Ala Phe Tyr Thr Thr Lys Asn Ile Ile Gly Thr | PND142 | 2 |
| SC | r-Asn Asn Thr Thr Arg Ser Ile His Ile Gly Pro Gly Arg Ala PheTyr Ala Thr Gly Asp Ile Ile Gly Asp Ile | PND-SC | 3 |
| IIIB | r-Asn Asn Thr Arg Lys Ser Ile Arg Ile Gln Arg Gly Pro Gly Arg Ala Phe Val Thr Ile Gly Lys Ile Gly Asn | PND135 | 4 |

| | | | |
|---|---|---|---|
| IIIB | r-Arg Ile Gln Arg Gly Pro Gly Arg Ala Phe Val Thr Ile Gly Lys Ile Gly Asn | PND135-18 | 5 |
| IIIB | r-Arg Ile Gln Arg Gly Pro Gly Arg Phe Val Thr | PND135-12 | 6 |
| MN | r-His Ile Gly Pro Gly Arg Ala Phe | PND-MN8 | 7 |
| MN | r-Gly Pro Gly Arg Ala Phe | PND-MN6 | 8 |
| LAV-1 | r-Ile Gln Arg Gly Pro Gly Arg Ala Phe | PND-LAV-1 | 9 |
| SF2 | r-Ile Tyr Ile Gly Pro Gly Arg Ala Phe | PND-SF2 | 10 |
| NY5 | r-Ile Ala Ile Gly Pro Gly Arg Thr Leu | PND-NY5 | 11 |
| CDC4 | r-Val Thr Leu Gly Pro Gly Arg Val Trp | PND-CDC4 | 12 |
| RF | r-Ile Thr Lys Gly Pro Gly Arg Val Ile | PND-RF | 13 |
| ELI | r-Thr Pro Ile Gly Leu Gly Gln Ser Leu | PND-ELI | 14 |
| Z6 | r-Thr Pro Ile Gly Leu Gly Gln Ala Leu | PND-Z6 | 15 |
| MAL | r-Ile His Phe Gly Pro Gly Gln Ala Leu | PND-MAL | 16 |
| Z3 | r-Ile Arg Ile Gly Pro Gly Lys Val Phe | PND-Z3 | 17 |

This list is not an exhaustive list of possible PND sequences. Rather, it is provided as a suggestive and

illustrative guide as to useful PND primary sequences. Therefore, peptides conjugated as herein described to form the immunogen of this invention are any of the tabulated peptides or immunologically-equivalent variants on the theme suggested by these peptidyl sequences. The nature of the variations is considered next.

The primary sequence of this HIV PND appears to have a conserved core amino acid sequence, comprised by the tetramer sequence -Gly Pro Gly Arg-, (SEQ. ID NO: 18) with increacing divergence on either side of this sequence among HIV isolates. Some isolates have sequences that diverge even within the tetramer, having -Gly Pro Gly Lys-, (SEQ. ID NO: 19) -Gly Pro Gly Gln-, (SEQ. ID. NO: 20) and even -Gly Leu Gly Gln- (SEQ. ID. NO. 21) core sequences. All of these possible sequences come within the scope of this disclosure as being peptide sequences that are advantageous for conjugation according to this invention.

The length of the peptide is a significant factor in promoting cross reactive immune responses. That is, an immune response raised against a given peptidyl epitope may recognize similar epitopes from the same or different HIV isolate based on the number of amino acids in the epitope over and above the critical neutralizing epitope. In addition, the length of the peptide is also responsible for determining the probability of exposure to the immune system of the determinant responsible for generating an HIV-neutralizing response.

In order to maximize the probability of relevant epitope presentation, chemistry was developed whereby the PND peptides may be locked into a given three-dimensional configuration. It is known that the 41-amino-acid PND of the HIV IIIB isolate, represented above, is configured as a loop by the presence of the cysteine-to-cysteine disulfide bond. Disulfides, however, may be labile under certain conditions and therefore may allow the loop to open and the peptide to exist in a linear form. Therefore, in addition to linear peptides, disulfide-bonded cyclic peptides and novel HIV PND peptides having nonlabile cyclic structures disclosed herein but separately claimed as free peptides in co-pending US application serial Nos. 07/715,127, 555,112 and 555,227, corresponding to EP-A-0467701, EP-A-0471453 and EP-A-0467699, may all be utilized as the PEP component in the formation of the conjugates of this invention.

The peptides that may be used in formation of these conjugates may be derived as fragments of natural proteins (gp120 for example), by recombinant expression of portions thereof, or by chemical synthesis according to known methods in the art. In addition, novel cyclic PNDs may be prepared synthetically according to the processes herein described. The sequences may contain both natural L-amino acids, or unusual or D-amino acids. In addition, the conjugation chemistry is sufficiently flexible so that the appropriate choice of peptide derivatization reagents allows for successful conjugation.

Synthetic peptides have been prepared by a number of strategies conducted either in solution or on solid supports. Excellent texts covering the basic principles and techniques are: Principles of Peptide Synthesis, Bodanszky. M., Springer-Verlag (1984); Solid Phase Peptide Synthesis, Stewart J. M., Young, J. D., Pierce Chemical Company (2nd. ed. 1984); and The Peptides, Gross, E., Meienhofer, J., Academic Press, Inc., (1979). The processes described herein, however, are not limited to the disclosure of these texts.

Synthetic cyclic peptides may be prepared in two phases. First, the linear peptide may be synthesized on a Milligen 9050 peptide or an ABI 431A synthesizer using 9-fluorenylmethyloxy-carbonyl (Fmoc) chemistry and side-chain-protected Fmoc-amino acid pentafluorophenyl esters which are known reagents or using derivatized Wang resin, Fmoc chemistry, and side-chain protected Fmoc-amino acid symmetrical anhydrides, prepared in situ, as reagents.

Second, the linear peptide may be cyclized, either in solution or with the peptide still attached to the solid phase resin. Cyclization may be accomplished by any technique known in the art, which may comprise, for example: a) incorporating cysteine residues into the linear peptide on either end of the sequence which is to form the loop and allowing disulfide bond formation under oxidizing conditions known in the art; b) preparing a cysteine containing peptide as in (a) but retaining the cysteines as free sulfhydryls (or as Acm protected thiols which are deprotected to the free sulfhydryls) and treating the peptide with o-xylylene dibromide or similar reagent, such as the diiodide, dichloride, or a dihalogenated straight or branched chain lower alkyl having between two and eight carbon atoms; such reagents react with the sulfur atoms of the cysteines to form a cyclic structure containing two nonlabile thioether bonds to the benzene or the alkyl; c) allowing a free group on one side of the loop amino acids to become amide bonded to a free carboxyl group on the other side of the loop amino acids through DPPA, BOP, or similar reagent mediated peptide bond formation. Each of these strategies is taken up in more detail below, after presentation of a generalized description of the cyclic peptides produced by these methods.

Thus, without limiting the conjugate invention to the following peptides or methods of producing them, the PND peptides which may be conjugated after removal of appropriate protecting groups as necessary,

according to this invention include those represented by the structure PEP, which includes the linear peptides of Table II above and cyclic peptides: (SEQ. ID. NO: 18)

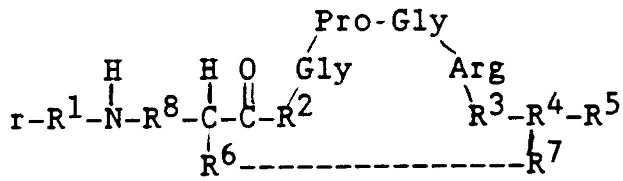

wherein:

- r is the position of linkage between PEP and OMPC, optionally comprising a marker amino acid, if $R^1$ is not a marker amino acid;
- $R^1$ is:
  - a) a bond, or
  - b) an amino acid or a peptide of 2 to 5 amino acids, optionally including a marker amino acid which migrates at a position in the amino acid analysis spectrum which is isolated from the signal of the 20 naturally occuring amino acids; preferably norleucine, gamma aminobutyric acid, $\beta$-alanine, or ornithine;
- $R^2$ is :
  - a) either a bond, an amino acid, or a peptide of 2 up to 17 amino acids, or
  - b) a peptide of between 2 to 17 amino acids;
- $R^3$ is:
  - a) either a bond, an amino acid, or a peptide of 2 up to 17 amino acids or
  - b) a peptide of between 2 to 17 amino acids;

$R^2$ and $R^3$ cannot both be option (a);

- $R^4$ is:
  - a) -NH-CH-CO-, with $R^7$ bonded to the methine carbon, if $R^7$ is $R^8$, or
  - b) a bond from $R^3$ to $R^7$ and $R^5$, if $R^7$ is carbonyl or $-COCH_2CH_2CH(CONH_2)NHCO-$;
- $R^5$ is:
  - a) an amino acid or a peptide of two to five amino acids, optionally including a marker amino acid,
  - b) -OH,
  - c) -COOH,
  - d) $-CONH_2$,
  - e) $-NH_2$, or
  - f) -absent;
- $R^6$ is:
  - a) an amino acid side chain, selected from the side chain of any of the common L or D amino acids, (see table of Definitios and Abbreviations), if the optional bond (--------) to $R^7$ is absent,
  - b) $-R^8$-S-S-, or $-R^8-S-R^8-R^9-R^8-S-$, if $R^7$ is $R^8$, or
  - c) $-R^8$-NH- if $R^7$ is

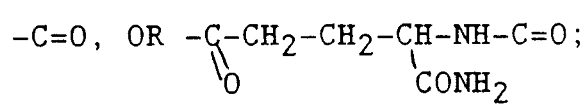

- $R^7$ is:
  - a) $-R^8-$,
  - b) -C = O, or
  - c)

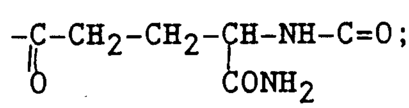

$R^8$ is a bond or lower alkylene of between one and eight carbons;
$R^9$ is:
a) $R^{10}$, or
b) xylylene
$R^{10}$ is:
a) lower alkylene, or
b) $-CH_2-O-CH_2-$; and

every occurrence of a variable is independent of every other occurrence of the same variable. When a peptide has been synthesized with a protected amino terminal amino acid, the an amino terminal protecting group such as benzyloxy carbonyl (Z) for protecting amines, or acetamidomethyl (Acm) for protecting sulfhydryls, may be removed according to methods known in the art and exemplified herein. The deprotected group thus revealed may be utilized in covalent bond formation, through the linker r, to the immunogenic protein.

Hereinafter, amino acids $-R^2$-Gly Pro Gly Arg-$R^3$-, (SEQ. ID. NO: 18) which form the "core" of the PND peptides, and go toward formation of the loop of a cyclic peptide, will be referred to as loop or core amino acids. When the optional bond between $R^6$ and $R^7$ is absent however, the structure, PEP, is linear, and encompasses all of the linear peptides of Table II.

Whether the peptide is linear or cyclic, the amino acid sequences comprising $R^2$ and $R^3$ of PEP may be any combination of amino acids, including sequences surrounding the core -Gly Pro Gly Arg-(SEQ. ID. NO: 18) tetramer in any of the sequences of Table II. Thus, the core amino acids represented by $-R^2$-Gly Pro Gly Arg-$R^3$- (SEQ. ID. NO: 18) may be further defined as having the core amino acid structure: (SEQ. ID. NO: 18)

$-X_nX_1X_2$-Gly Pro Gly Arg-$X_3X_4X_m$-

wherein:
$X_1$ is a constituent of $R^2$ selected from:
a) serine,
b) proline,
c) arginine,
d) histidine,
e) glutamine, and
f) threonine;
$X_2$ is a constituent of $R^2$ selected from:
a) isoleucine,
b) arginine,
c) valine, and
d) methionine;
$X_n$ is a constituent of $R^2$ and is either a bond, an amino acid, or a peptide of up to 15 amino acids;
$X_3$ is a constituent of $R^3$ selected from:
a) alanine,
b) arginine, and
c) valine;
$X_4$ is a constituent of $R^3$ and is selected from:
a) phenylalanine,
b) isoleucine,
c) valine, and
d) leucine;
$X_m$ is a constituent of $R^3$ and is a bond, an amino acid, or a peptide of up to 15 amino acids.

The cyclic peptides may be disulfide bonded structures or a cycle formed through a nonlabile bond or structure. The term "nonlabile bond" means a covalent linkage, other than a disulfide bond. Examples of such nonlabile bonds are amide and thioether bonds as disclosed in co-pending applications USSN 555,112 and 555,227, corresponding to EP-A-0471453 and EP-A-0467699. These covalent linkages may be through a bridge structure, such as xylylene, through a lower alkyl, through $-CH_2-O-CH_2$, or through an amino acid amide-bonded bridge. By altering the bridge structure and/or the number and combination of amino acids included in the peptide, the conformation of the loop structure of the cycle may be optimized, allowing for fine-tuning of the PND epitope presented to the immune system. For example, use of an o-xylylene bridge generates a "tighter" loop structure than when, for example, an eight carbon straight chain lower alkyl is used as the bridge. Thus, the conjugates of this invention are useful both as reagents to analyze the

structure-function relationship of the PND epitope in raising anti-peptide, anti-HIV, HIV-neutralizing, and anti-AIDS immune responses in mammals, and as components for formulation of anti-HIV disease, including AIDS, vaccines.

Synthetic products obtained may be characterized by fast-atom-bombardment mass spectrometry [FAB-MS], reverse phase HPLC, amino acid analysis, or nuclear magnetic resonance spectroscopy (NMR).

a. Cyclic Peptides through Disulfide-Bonded Cysteines:

Peptides containing cysteine residues on either side of the loop amino acids may be cyclized under oxidizing conditions to the disulfide-bonded cycles. Methods for achieving disulfide bonding are known in the art. An example of disulfide bonded peptides useful in this invention is given infra in Example 10, wherein cPND4 is produced and Example 18 wherein cPND33 is produced. In Example 10, a process utilizing the Acm derivative of cysteine to generate disulfide bonded cPNDs is used, but other processes are equally applicable. In Example 18, the peptide containg two sulfhydryls is oxidized in dilute acid. The disulfide bonded peptides are preferred in the instant invention.

Thus, in a preferred embodiment of this invention, the peptide has the structure:

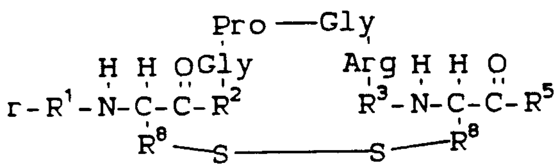

or pharmaceutically acceptable salts thereof, wherein:

r is:

a) hydrogen,

b)

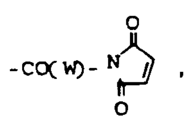

wherein W is preferably $-(CH_2)_2-$ or $-(CH_2)_3-$ or $R^6$, where $R^6$ is

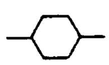 , 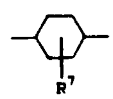 , 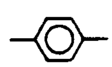 or 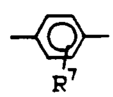

wherein $R^7$ is lower alkyl, lower alkoxy, or halo;

$R^1$ is:

a) a bond, or

b) a peptide of 1 to 5 amino acids, optionally including a marker amino acid;

$R^2$ is : a peptide of 3 to 10 amino acids

$R^3$ is: a peptide of 3 to 10 amino acids

-GPGR- is the tetramer -GlyProGlyArg-;

$R^5$ is:

a) -OH,

b) an amino acid or a peptide of 2 to 5 amino acids, optionally including a marker amino acid, or

c) $-NH_2$;

$R^8$ is lower alkylene of between one and eight carbons.

Lower alkylene consists of straight or branched chain alkylenes having from one to eight carbons unless otherwise specified. Hereinafter, amino acids $-R^2$-Gly Pro Gly Arg-$R^3$-, (SEQ. ID. NO: 18) which go toward formation of the loop of a cyclic peptide, will be referred to as loop amino acids.

In one embodiment of the invention, the cyclic peptide having the structure:

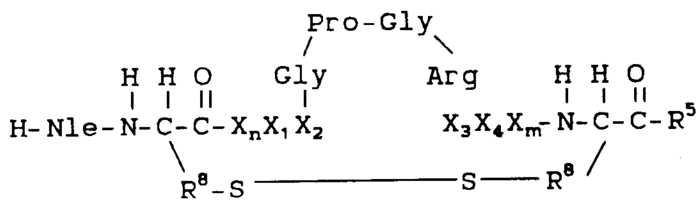

is prepared by cyclizing a linear peptide having the structure:

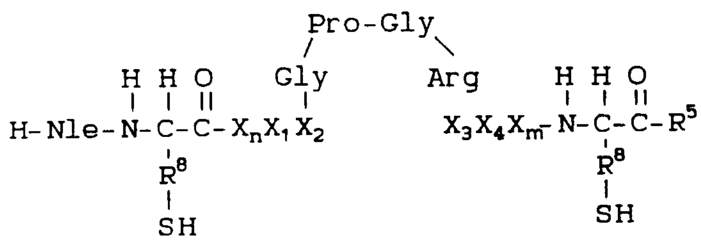

wherein:

$X_1$ is a constituent of $R^2$ selected from:
- a) serine,
- b) proline,
- c) arginine,
- d) histidine,
- e) glutamine, which is preferred, and
- f) threonine,

$X_2$ is a constituent of $R^2$ selected from:
- a) isoleucine, which is most preferred,
- b) arginine, which is preferred,
- c) valine, and
- d) methionine;

$X_n$ is a constituent of $R^2$ and is an amino acid or a peptide of up to 8 amino acids;

$X_3$ is a constituent of $R^3$ selected from:
- a) alanine,
- b) arginine, and
- c) valine;

$X_4$ is a constituent of $R^3$ and is selected from:
- a) phenylalanine,
- b) isoleucine,
- c) valine, and
- d) leucine;

$X_m$ is a constituent of $R^3$ and is an amino acid or a peptide of up to 8 amino acids.

$X_2$ is preferably Isoleucine.

The disulfide bonded cyclic peptides used in this invention may be prepared in essentially two phases: First the linear peptide is synthesized on a Milligen 9050 or an ABI-431A peptide synthesizer using 9-fluorenyl-methyloxycarbonyl (Fmoc) chemistry and appropriately side-chain protected Fmoc-amino acid pentafluoro-phenyl esters as reagents or using derivatized Wang resin, Fmoc chemistry, and side-chain protected Fmoc-amino acid symmetrical anhydrides, prepared in situ, as reagents.

Second, the linear peptide is cyclized, either in solution or with the peptide still attached to the solid phase resin by incorporating cysteine residues into the linear peptide at either end of the sequence which is to form the loop, and oxidizing these to the disulfide. In a preferred embodiment, cyclization is accomplished by exposure of the peptide to (a) $H_2O_2$, (b) atmospheric oxygen, (c) aqueous $CH_3CN$ containing about 0.1 - 0.5% TFA, or (d) about 0.1M ferricyanide. The preferred method is exposure to atmospheric oxygen.

Products obtained may be characterized by fast atom bombardment-mass spectrometry [FAB-MS], reverse phase HPLC, amino acid analysis or nuclear magnetic resonance spectroscopy (NMR).

Thus, the peptides useful in this invention may be prepared as further described below in (i) and (ii):

i. Peptide Cyclization in the Solid State: A linear peptide containing $C^1$ and $C^2$ on either side of the loop amino acids, where $C^1$ and $C^2$ are both cysteine or another amino acid containing free sulfhydryl groups in the side chain, is prepared according to known synthetic procedures (see discussion supra). In the completed cyclic PND, the sulfhydryl containing side chains, ($-R^8$-SH), go toward making up the $-R^8$-S- groups of the completed cyclic HIV PND structure shown above. Amino acids to be incorporated which have reactive side chains (R groups) are used in an appropriately R-group protected form. For example, histidine is triphenylmethyl (Trt), or Boc protected, and arginine is 4-methoxy-2,3,6-trimethylphenyl sulfonyl (Mtr) protected.

Preferably, a resin is purchased with $C^2$ in its Acm protected form already attached to the resin, for example, Fmoc-L-Cys(Acm)-O-Wang resin. The cysteine incorporated at the amino terminal side of the loop amino acids, $C^1$, may also be the Acm derivative. Either $C^1$ or $C^2$ may be bound to additional amino acids, $R^1$ or $R^5$ respectively, which may be utilized in the formation of conjugates with carrier molecules or may serve as marker amino acids for subsequent amino acid analysis, such as when norleucine or ornithine is used.

The sulfur of the acetamidomethylated cysteines are reacted, at room temperature for about 15 hours in a solvent compatible with the resin, as a 1-50% concentration of an organic acid, preferably about 10% acetic acid in anhydrous dimethylformamide (DMF), with about a four fold molar excess of a heavy metal salt, such as mercuric acetate [$Hg(OAc)_2$] for each Acm group. The resulting heavy metal thioether, for example the mercuric acetate thioether of the peptide, PEP(S-HgOAc), is then washed and dried. Addition of excess hydrogen sulfide in DMF yields insoluble metal sulfide, e.g. mercuric sulfide (HgS), and the peptide with free sulfhydryl groups. The free sulfhydryls are then oxidized by one of the aforementioned methods. Alternatively, the Acm protected thiols may be converted directly to the cyclic disulfide by treatment with iodine in a methanol/DMF solvent.

ii. Cyclization of Peptides in Solution:

Essentially the same process described above for solid state cyclization applies with two main variants: If the peptide is cleaved (95% TFA/4% ethanedithiol/1% thioanisole) from a pepsyn KA resin, acid labile side chain protecting groups are also removed, including Cys(Trt) which provides the necessary free -SH function. If however, Cys(Acm) protection is used, then mercuric acetate/hydrogen sulfide cleavage to the free -SH group is required as an independent procedure, with the linear peptide either on or off the resin.

One method however, is the use of Cys(Acm) protection and Sasrin or Pepsyn KH resin, and cleavage of the linear, fully protected peptide from the resin with 1% $TFA/CH_2Cl_2$. Mercuric acetate/ hydrogen sulphide then selectively converts Cys(Acm) to the free -SH group, and cyclization is effected on the otherwise protected peptide. At this point, the peptide may be maleimidated in situ , selectively on the N-terminus. Acid labile side chain protecting groups are cleaved with 98% TFA/2% thioanisole, and the cyclic peptide is isolated by HPLC. The preferred method, however, is to cleave the peptide from the resin, and allow cyclization by one of the aforementioned methods. The most preferred method is to allow air oxidation for about one to fifty hours of between 10 and 40 °C.

Thus, in a particularly preferred embodiment of this invention, a peptide (CPND 33) having the structure: (SEQ. ID. NO: 22)

```
H-Nle Cys Tyr Asn Lys Arg Lys Arg Ile His Ile Gly Pro
       |            Gly Arg Ala Phe Tyr Thr Thr Lys Asn
       |            Ile Ile Gly Cys-OH
       |                         |
       S ----------------------- S
```

is conjugated to MIEP through either the amino terminal Nle or one of the internal lysines to generate one or a mixture of all of the structures:

e-1)

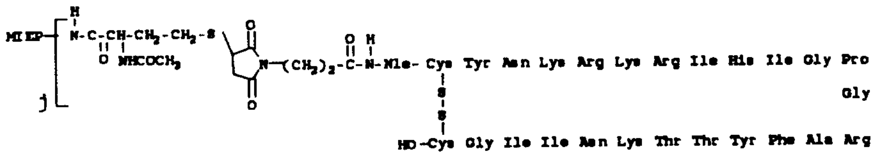

e-2)

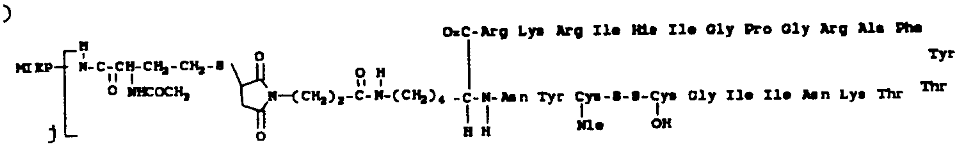

e-3)

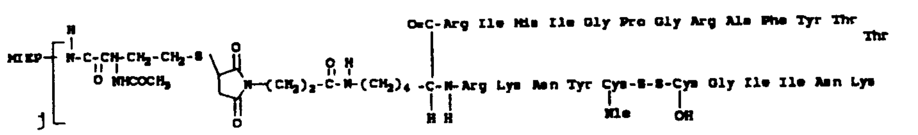

or

e-4)

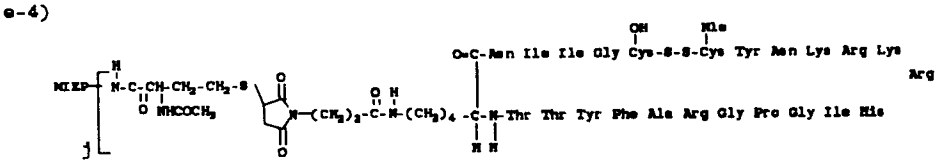

wherein j is the percentage by mass of peptide in the conjugate, and is preferably between 1% and 50% of the total protein mass in the conjugate.

b. Cyclic Peptides through Thioether Linkage to o-Xylylene or Lower Alkyls:

i. Peptide Cyclization in the Solid State: A linear peptide containing $C^1$ and $C^2$ on either side of the loop amino acids, where $C^1$ and $C^2$ are both cysteine or another sulfhydryl containing amino acid, is prepared according to known synthetic procedures (see discussion supra). In the completed cylic PND, $C^1$ and $C^2$ become part of the $R^6$ and $R^7$ groups of the PEP structure shown above. Amino acids to be incorporated which have reactive side chains (R groups) are used in an appropriately R-group-protected form. For example, histidine is triphenylmethyl- (Trt) protected, arginine may be 4-methoxy-2,3,6-trimethylphenyl sulfonyl (Mtr) protected. [Principles of Peptide Synthesis, Bodanszky. M., Springer-Verlag (1984); Solid Phase Peptide Synthesis, Stewart J. M., Young, J. D., Pierce Chemical Company (2nd. ed. 1984); and The Peptides, Gross, E., Meienhofer, J., Academic Press, Inc., (1979)].

Preferably, a resin is purchased with $C^2$ in its Acm-protected form already attached to the resin, for example, Fmoc-L-Cys(Acm)-O-Wang resin. The cysteine incorporated at the amino terminal side of the loop amino acids, $C^1$, may also be the Acm derivative. Either $C^1$ or $C^2$ may be bound to additional amino acids, $R^1$ or $R^5$ respectively, which may be utilized in the formation of conjugates with carrier molecules or may serve as marker amino acids for subsequent amino acid analysis, such as when norleucine or ornithine is used.

The sulfur of the acetamidomethylated cysteines is reacted, at room temperature for about 15 hours in

a solvent compatible with the resin, such as 10% acetic acid in anhydrous dimethylformamide (DMF), with about a four-fold molar excess of a heavy metal salt, such as mercuric acetate [$Hg(OAc)_2$] for each Acm group. The resulting heavy metal thioether, for example the mercuric acetate thioether of the peptide, PEP-(S-HgOAc), is then washed and dried. Addition of excess hydrogen sulfide in DMF yields insoluble metal sulfide, e.g., mercuric sulfide (HgS), and the peptide with free sulfhydryl groups.

A mixture of about an equimolar amount, as compared with peptide, of o-xylylene dibromide or dichloride, a dibrominated or dichlorinated lower alkyl, 1,3-dihalogenenated -CH-O-CH-, or similar reagent which will provide a desirable bridge length, is added to the derivatized resin. A large excess of tertiary amine, preferably triethylamine ($NEt_3$) in DMF is added slowly. The reaction with the bis-sulfhydryl peptide moiety is allowed to proceed for about sixteen hours at room temperature, yielding the bridge group derivatized cyclic peptide bound to resin. Deprotection of acid sensitive side chain protecting groups and cleavage from the resin is achieved by treatment with 95% trifluoroacetic acid (TFA) in the presence of 4% 1,2-ethanedithiol and 1% thioanisole. The dissolved cyclic peptide may then be separated from the resin by filtration. The filtrate is evaporated and the crude residual product is purified by high performance liquid chromatography (HPLC) according to known methods, for example by reverse phase HPLC.

ii. Cyclization of Peptides in Solution:

Essentially the same process described above for solid state cyclization applies with two main variants: If the peptide is cleaved (95% TFA/4% ethanedithiol/1% thioanisole) from a pepsyn KA resin, acid labile side chain protecting groups are also removed, including Cys(Trt) which provides the necessary free -SH function. If however, Cys(Acm) protection is used, then mercuric acetate/hydrogen sulfide cleavage to the free -SH group is required as an independent procedure, with the linear peptide either on or off the resin.

The preferred method however, is the use of Cys(Acm) protection and Sasrin or Pepsyn KH resin, and cleavage of the linear, fully protected peptide from the resin with 1% TFA/$CH_2Cl_2$. Mercuric acetate/hydrogen sulphide then selectively converts Cys(Acm) to the free -SH group, and cyclization is effected on the otherwise protected peptide. Acid labile side chain protecting groups are cleaved with 95% TFA/4% ethanedithiol/1% thioanisole, and the cyclic peptide is isolated by HPLC.

Removal of excess reagents, such as unreacted xylylene dibromide, prior to acid cleavage of the protecting groups is conveniently achieved by, for example, a step gradient reverse phase HPLC run prior to more selective gradient elution.

Cyclic HIV PND peptides prepared according to the method of this subsection include, but are not limited to, the sample cPNDs represented below. The methods of this subsection are generally applicable to small peptides, and particularly applicable to peptides of between 5 and 30 amino acids. An optimal ring size may include between 5 and 10 amino acids, including the -Gly-Pro-Gly- trimer, and this ring size is easily maintained by production of cycles from linear peptides having the appropriate number and combination of amino acids.

Representative peptides resulting from the process described in this subsection b. parts (i). and (ii) are disclosed inapplication U.S.S.N. 555,227, corresponding to EP-A-0467699.
The conjugate invention should, however, not be construed as being limited to use those particular embodiments of HIV cyclic PND peptides. Other linear HIV PND peptide sequences may be cyclized in essentially the same fashion used to provide those peptides. Series of peptides having divergent primary sequences could be generated and would be beneficial in this invention as long as they continue to elicit an anti-peptide, anti-HIV, or HIV-neutralizing immune response.

c. Cyclization through Amide Bond Formation:

Novel amide bonded cyclic HIV PND peptides may be prepared for conjugation in essentially two phases: First, the linear peptide is prepared, for example on an ABI-431A peptide synthesizer, by known solid phase peptide synthetic chemistry, for example using Fmoc chemistry and appropriately side-chain protected Fmoc-amino acids as reagents.

Second, the linear peptide is cleaved from the resin and cyclized in solution by allowing the free amino terminus of the peptide, the free amino group of an amino terminal isoglutamine, or a free $\epsilon$-amino or $\alpha$-amino group of a lysine on one side of the loop amino acids to be amide bonded to a free carboxyl group on the carboxy-terminal side of the loop amino acids through DPPA, BOP, or similar reagent mediated peptide bond formation.

Products obtained may be characterized by fast atom bombardment-mass spectrometry [FAB-MS], reverse phase HPLC, amino acid analysis, or nuclear magnetic resonance spectroscopy (NMR).

Thus, preferred embodiments of this invention are conjugates having covalent linkages from MIEP to an amide bonded cyclic HIV PND, prepared as described hereinabove. Where the PND is from a predominant isolate, such as the HIV IIIB or the HIV MN isolate, a conjugate vaccine, or a mixture of such conjugate vaccines is highly advantageous for prophylaxis or treatment of AIDS or ARC. Examples of such preferred embodiments having the structure:

a)

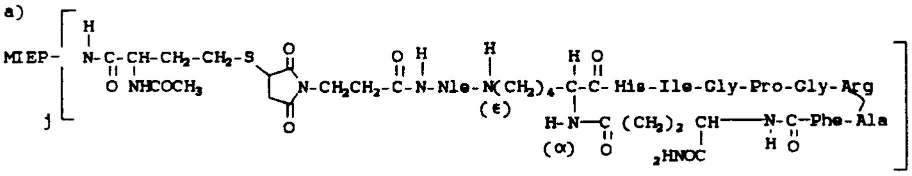

b)

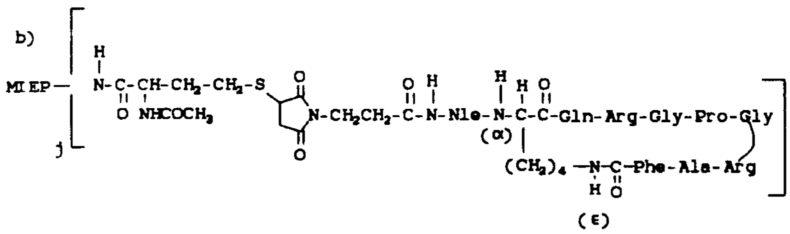

or pharmaceutically acceptable salts thereof, wherein:

- j is the percentage by mass of peptide in the conjugate, and is preferably between 1% and 50% of the total protein mass in the conjugate;

are useful for inducing anti-peptide immune responses in mammals, for inducing HIV-neutralizing antibodies in mammals, for formulating vaccines to prevent HIV-disease or infection, or for treating humans afflicted with HIV-disease or infection, including AIDS and ARC.

EXAMPLE 1

## Preparation of Neisseria meningitidis B11 Serotype 2 OMPC

### A. Fermentation

1. Neisseria meningitidis Group B11

A tube containing the lyophilized culture of Neisseria meningitidis (obtained from Dr. M. Artenstein, Walter Reed Army Institute of Research (WRAIR), Washington, D.C.) was opened and Eugonbroth (BBL) was added. The culture was streaked onto Mueller Hinton agar slants and incubated at 37°C with 5% $CO_2$ for 36 hours, at which time the growth was harvested into 10% skim milk medium (Difco), and aliquots were frozen at -70°C. The identity of the organism was confirmed by agglutination with specific antiserum supplied by WRAIR, and typing serum supplied by Difco.

A vial of the culture from the second passage was thawed and streaked onto 10 Columbia Sheep Blood agar plates (CBAB-BBL). The plates were incubated at 37°C with 5% $CO_2$ for 18 hours after which time the growth was harvested into 100 mL of 10% skim milk medium, aliquots were taken in 0.5 mL amounts and frozen at -70°C. The organism was positively identified by agglutination with specific antiserum, sugar fermentation and gram stain.

A vial of the culture from this passage was thawed, diluted with Mueller-Hinton Broth and streaked onto 40 Mueller-Hinton agar plates. The plates were incubated at 37°C with 6% $CO_2$ for 18 hours after which time the growth harvested into 17 mL of 10% skim milk medium, aliquotted in 0.3 mL amounts and frozen

at -70°C. The organism was positively identified by Gram stain, agglutination with specific antiserum and oxidase test.

2. Fermentation and collection of cell paste

a. Inoculum Development- The inoculum was grown from one frozen vial of Neisseria memingitidis Group B, B-11 from above (passage 4). Ten Mueller-Hinton agar slants were inoculated, and six were harvested approximately 18 hours later, and used as an inoculum for 3 250 mL flasks of Gotschlich's yeast dialysate medium at pH 6.35. The O.D.$_{660}$ was adjusted to 0.18 and incubated until the $OD_{660}$ was between 1 and 1.8. 1 mL of this culture was used to inoculate each of 5 2L. Erlenmeyer flasks (each containing 1 liter of medium; see below) and incubated at 37°C in a shaker at 200 rpm. The O.D. was monitored at hourly intervals following inoculation. 4 liters of broth culture, at an O.D.$_{660}$ of 1.28 resulted.

70 Liter Seed Fermenter- Approximately 4 liters of seed culture was used to inoculate a sterile 70-liter fermenter containing about 40 liters of complete production medium (see below). The conditions for the 70-liter fermentation included 37°C, 185 rpm with 10 liters/minute air sparging and constant pH control at about pH 7.0 for about 2 hours. For this batch, the final O.D.$_{660}$ was 0.732 after 2 hours.

800-Liter Production Fermenter

Approximately 40 liters of seed culture were used to inoculate a sterile 800 liter fermenter containing 568.2 liters of complete production medium (see below). The batch was incubated at 37°C, 100 rpm with 60 liters/minute air sparging and constant pH control at pH 7.0. For this batch, the final O.D. was 5.58 thirteen hours after inoculation.

3. Complete Medium for Erlenmeyer flasks and 70-and 800-liter fermenters

| **Fraction A** | |
|---|---|
| L-glutamic acid | 1.5 g/liter |
| NaCl | 6.0 g/liter |
| $Na_2HPO_4$.anhydrous | 2.5 g/liter |
| $NH_4Cl$ | 1.25 g/liter |
| KCl | 0.09 g/liter |
| L-cysteine HCl | 0.02 g/liter |

**Fraction B (Gotschlich's Yeast Dialysate):**

1280 g of Difco Yeast Extract was dissolved in 6.4 liters of distilled water. The solution was dialyzed in 2 Amicon DC-30 hollow fiber dialysis units with three H10SM cartridges. 384 g $MgSO_4$.7-$H_2O$ and 3200 g dextrose were dissolved in the dialysate and the total volume brought to 15 liters with distilled water. The pH was adjusted to 7.4 with NaOH, sterilized by passage through a 0.22 $\mu$ filter, and transferred to the fermenter containing Fraction A.

For the Erlenmeyer flasks: 1 liter of Fraction A and 25 mL of Fraction B were added and the pH was adjusted to 7.0-7.2 with NaOH.

For the 70 liter fermenter: 41.8 liters of Fraction A and 900 mL of Fraction B were added and the pH was adjusted to 7.0-7.2 with NaOH.

For the 800 liter fermenter: 553 liters of Fraction A and 15.0 liters of Fraction B were added and the pH was adjusted to 7.1-7.2 with NaOH.

d. Harvest and Inactivation

After the fermentation was completed, phenol was added in a separate vessel, to which the cell broth was then transferred, yielding a final phenol concentration of about 0.5%. The material was held a room temperature with gentle stirring until the culture was no longer viable (about 24 hours).

e. Centrifugation

After about 24 hours at 4°C, the 614.4 liters of inactivated culture fluid was centrifuged through Sharples continuous flow centrifuges. The weight of the cell paste after phenol treatment was 3.875 kg.

**B. OMPC Isolation**

Step 1. Concentration and diafiltration

The phenol inactivated culture was concentrated to about 30 liters and diafiltered in sterile distilled water using 0.L micro-hollow fiber filters (ENKA).

Step 2. Extraction

An equal volume of 2X TED buffer [0.1 M TRIS 0.01 M EDTA Buffer, pH 8.5, with 0.5% sodium deoxycholate] was added to the concentrated diafiltered cells. The suspension was transferred to a temperature regulated tank for OMPC extraction at 56 °C with agitation for 30 minutes.

The extract was centrifuged at about 18,000 rpm in a Sharples continuous flow centrifuge at a flow rate of about 80 mL/minute, at about 4°C. The viscous supernatant was then collected and stored at 4°C. The extracted cell pellets were reextracted in TED buffer as described above. The supernatants were pooled and stored at 4°C.

Step 3. Concentration by Ultrafiltration

The pooled extract was transferred to a temperature regulated vessel attached to AG-Tech 0.1 micron polysulfone filters. The temperature of the extract was held at 25°C in the vessel throughout the concentration process. The sample was concentrated tenfold at an average transmembrane pressure of between 11 and 24 psi.

Step 4. Collection and Washing of the OMPC

The retentate from Step 3 was centrifuged at about 160,000 x g (35,000 rpm) at about 70°C in a continuous flow centrifuge at a flow rate between 300 to 500 mL/minute, and the supernatant was discarded.

The OMPC pellet was suspended in TED Buffer (190 mL buffer; 20 mL/g pellet) Step 2 and Step 4 were repeated twice (skipping Step 3).

Step 5. Recovery of OMPC Product

The washed pellets from Step 4 were suspended in 100 mL distilled water with a glass rod and a Dounce homogenizer to insure complete suspension. The aqueous OMPC suspension was then filter sterilized by passage through a 0.22 μ filter, and the TED buffer was replaced with water by diafiltration against sterile distilled water using a 0.1 μ hollow fiber filter.

EXAMPLE 2

Cloning of Genomic DNA Encoding MIEP:

About 0.1 g of the phenol inactivated *N. meningitidis* cells (see Example 1) was placed in a fresh tube. The phenol inactivated cells were resuspended in 567 μL of TE buffer [10mM TRIS-HCl, 1mM EDTA, pH 8.0]. To the resuspended cells was added 30 μL of 10% SDS, and 3 μL of 20 mg/mL proteinase K (Sigma). The cells were mixed and incubated at 37°C for about 1 hour, after which 100 μL of 5 M NaCl was added and mixed thoroughly. 80 μL of 1% cetyltrimethylamonium bromide (CTAB) in 0.7 M NaCl was then added, mixed thoroughly, and incubated at 65°C for 10 minutes. An equal volume (about 0.7 to 0.8 mL) of chloroform/isoamyl alcohol (at a ratio of 24:1, respectively) was added, mixed thoroughly and centrifuged at about 10,000 x g for about 5 minutes. The aqueous (upper) phase was transferred to a new tube and the organic phase was discarded. An equal volume of phenol/chloroform/isoamyl alcohol (at a ratio of 25:24:1, respectively) was added to the aqueous phase, mixed thoroughly, and centrifuged at 10,000 x g for about 5 minutes. The aqueous phase (upper) was transferred to a new tube and 0.6 volumes (about 420 μL) of isopropyl alcohol was added, mixed thoroughly, and the precipitated DNA was pelletted by centrifugation at 10,000 x g for 10 minutes. The supernatant was discarded, and the pellet was washed with 70% ethanol.

The DNA pellet was dried and resuspended in 100 μL of TE buffer, and represents N. meningitidis genomic DNA.

Two DNA oligonucleotides were synthesized which correspond to the 5' end of the MIEP gene and to the 3' end of the MIEP gene [Murakami, E.C. et al., (1989), Infection and Immunity, 57, pp.2318-23]. The sequence of the DNA oligonucleotide specific for the 5' end of the MIEP gene was: 5'-ACTAGTTGCA ATGAAAAAAT CCCTG-3'; (SEQ. ID NO: 24) and for the 3' end of the MIEP gene was: 5'-GAATTCAGAT TAGGAATTTG TT-3' (SEQ. ID NO: 25). These DNA oligonucleotides were used as primers for polymerase chain reaction (PCR) amplification of the MIEP gene using 10 nanograms of N. meningitidis genomic DNA. The PCR amplification step was performed according to the procedures supplied by the manufacturer (Perkin Elmer).

The amplified MIEP DNA was then digested with the restriction endonucleases SpeI and EcoRI. The 1.3 kilobase (kb) DNA fragment, containing the complete coding region of MIEP, was isolated by electrophoresis on a 1.5% agarose gel, and recovered from the gel by electroelution [Current Protocols in Molecular Biology, (1987), Ausubel, R.M., Brent, R., Kingston, R.E., Moore, D.D., Smith, J.A., Seidman, J.G. and Struhl, K., eds., Greene Publishing Assoc.]

The plasmid vector pUC-19 was digested with SpeI and EcoRI. The gel purified SpeI-EcoRI MIEP DNA was ligated into the SpeI-EcoRI pUC-19 vector and was used to transform E. coli strain DH5. Transformants containing the pUC-19 vector with the 1.3 kbp MIEP DNA were identified by restriction endonuclease mapping, and the MIEP DNA was sequenced to ensure its identity.

## EXAMPLE 3

### Construction of the pcI/I.Gal10p(B)ADHI$_t$ vector:

The Gal 10 promoter was isolated from plasmid YEp52 [Broach, et al., (1983) in Experimental Manipulation of Gene Expression, Inouye, M(Ed) Academic Press pp. 83-117] by gel purifying the 0.5 kilobase pair (Kbp) fragment obtained after cleavage with Sau 3A and Hind III. The ADHI terminater was isolated from vector pGAP.tADH2 [Kniskern, et al., 91986), Gene, 46, pp. 135-141] by gel purifying the 0.35 Kbp fragment obtained by cleavage with Hind III and SpeI. The two fragments were ligated with T4 DNA ligase to the gel purified puc18ΔHind III vector (the Hind III site was eliminated by digesting pUC18 with Hind III, blunt-ending with the Klenow fragment of E. coli DNA polymerase I, and ligating with T4 DNA ligase) which had been digested with BamHI and SphI to create the parental vector pGal1o-tADHI. This has a unique Hind III cloning site at the Gal10p.ADHI$_t$ junction.

The unique Hind III cloning site of pGal10.tADHI was changed to a unique BamHI cloning site by digesting pGal10.tADHI with Hind III, gel purifying the cut DNA, and ligating, using T4 DNA ligase, to the following Hind III-BamHI linker:

```
5'-AGCTCGGATCCG-3'
      3'-GCCTAGGCTCGA-5'.
```

The resulting plasmid, pGal10(B)tADHI, has deleted the Hind III site and generated a unique BamHI cloning site.

The Gal10p.tADHI fragment was isolated from pGal10(B)tADHI by digestion with SmaI and SphI, blunt-ended with T4 DNA polymerase, and gel purified. The yeast shuttle vector pCI/I [Brake et al., (1984), Proc. Nat'l. Acad. Sci. USA, 81, pp.4642-4646] was digested with SphI, blunt-ended with T4 DNA polymerase, anpurified. This fragment was ligated to the vector with T4 DNA ligase. The ligation reaction mixture was then used to transform E. coli HBI01 cells to ampicillin resistance, and transformants were screened by hybridization to a single strand of the $^{32}$P-labelled HindIII BamHI linker. The new vector construction, pcilI.Gal10p(B)ADHI$_t$ was confirmed by digestion with HindIII and BamHI.

## EXAMPLE 4

### Construction of a Yeast MIEP Expression Vector with MIEP + Leader DNA Sequences

A DNA fragment containing the complete coding region of MIEP was generated by digestion of pUC19.MIEP #7 with SpeI and EcoRI, gel purification of the MIEP DNA, and blunt-ended with T4 DNA

polymerase.

The yeast internal expression vector pCI/I.Gal10p(B)$ADHI_t$ was disgested with Bam HI, dephosphorylated with calf intestinal alkaline phosphatase, and blunt-ended with T4 DNA polymerase. The DNA was gel purified to remove uncut vector.

The 1.1 Kbp blunt-ended fragment of MIEP was ligated to the blunt-ended pcI/I.Gal10p(B)$ADHI_t$ vector, and the ligation reaction mixture was used to transform competent E. coli DH5 cells to ampicillin resistance. Transformants were screened by hybridization to a $^{32}$P-labelled DNA oilgoncleotide:

5'... AAGCTCGGAT CCTAGTTGCA ATG...3', (SEQ. ID NO: 27) which was designed to be homologous with sequences overlapping the MIEP-vector junction. Preparations of DNA were made from hybridization positive transformants and digested with KnpI and SalI to verify that the MIEP fragment was in the correct orientation for expression from the Gal10 promoter. Further confirmation of the DNA construction was obtained by dideoxy sequencing from the Gal10 promoter into the MIEP coding region.

Expression of MIEP by the transformants was detected by Western blot analysis. Recombinant MIEP produced in the transformants comigrated on polyacrylamide gels with MIEP purified from OMPC vesicles, and was immunologically reactive with antibodies specific for MIEP.

## EXAMPLE 5

### Construction of yeast MIEP expression vector with a 5'-Modified MIEP DNA Sequence.

A DNA oligonucleotide containing a HindIII site, a conserved yeast 5' nontranslated leader (NTL), a methionine start codon (ATG), the first 89 codons of the mature MIEP (beginning with Asp at position +20) and a KpnI site (at position +89) was generated using the polymerase chain reaction (PCR) technique. The PCR was performed as specified by the manufacturer (Perkin Elmer Cetus) using the plasmid pUC19MIEP42#7 as the template and the following DNA oligomers as primers:

$^{5'}$CTAAGCTTAA CAAAATGGAC GTTACCTTGT ACGGTACAAT T$^{3'}$, (SEQ. ID NO: 28) and

$^{5'}$ACGGTACCGA AGCCGCCTTT CAAG$^{3'}$ (SEQ. ID. NO: 29).

To remove the 5' region of the MIEP clone, plasmid pUC19MIEP42#7 was digested with KpnI and HindIII and the 3.4 Kbp vector fragment was agarose gel purified. The 280 bp PCR fragment was digested with KpnI and HindIII, agarose gel purified, and ligated with the 3.4 Kbp vector fragment. Transformants of E. coli HBl01 (BRL) were screened by DNA oilgonucleotide hybridization and the DNA from positive transformants was analyzed by restriction enzyme digestion. To ensure that no mutations were introduced during the PCR step, the 280 bp PCR generated DNA of the positive transformants was sequenced. The resulting plasmid contains a HindIII - EcoRI insert consisting of a yeast NTL, ATG codon, and the entire open reading frame (ORF) of MIEP beginning at the Asp codon (amino acid +20).

The yeast MIEP expression vectors were constructed as follows. The pGAL10/pcl/l and pGAP/pCI/I vectors [Vlasuk, G.P., et al., (1989) J.B.C., 264, pp.12,106-12,112] were digested with BamHI, flush-ended with the Klenow fragment of DNA polymerase I, and dephosphorylated with calf intestinal alkaline phosphatase. These linear vectors were ligated with the Klenow treated and gel purified HindIII - EcoRI fragment described above, which contains the yeast NTL, ATG and ORF of MIEP are forming pGal10/pcl/MIEP and pGAP/pGAP/pCI/MIEP.

Saccharomyces cerevisiae strain U9 (gal10pgal4-) were transformed with plasmid pGal10/p/pCI/MIEP. Recombinant clones were isolated and examined for expression of MIEP. Clones were grown at 37°C with shaking in synthetic medium (leu-) containing 2% glucose (w/v) to an $O.D._{660}$ of about 6.0. Galactose was then added to 2% (w/v) to induce expression of MIEP from the Gal10 promoter. The cells were grown for an additional 45 hours following galactose induction to an $O.D._{600}$ of about 9.0. The cells were then harvested by centrifugation. The cell pellet was washed with distilled water and frozen.

### Western Blot For Recombinant MIEP:

To determine whether the yeast was expressing MIEP, Western blot analysis was done. Twelve percent, 1 mm, 10 to 15 well Novex Laemmli gels are used. The yeast cells were broken in $H_2O$ using glass beads (sodium dodecylsulfate (SDS) may be used at 2% during the breaking process). Cell debris was removed by centrifugation for 1 minute at 10,000 x g.

The supernatant was mixed with sample running buffer, as described for polyacrylamide gel purification of MIEP. The samples were run at 35 mA, using OMPC as a reference control, until the dye part leaves the gel.

Proteins were transferred onto 0.45 $\mu$ pore size nitrocellulose paper, using a NOVEX transfer apparatus.

After transfer the nitrocellulose paper was blocked with 5% bovine serum albumin in phosphate buffered saline for 1 hour, after which 15 mL of a 1:1000 dilution of rabbit anti-MIEP (generated from gel purified MIEP using standard procedures) was added. After overnight incubation at room temperature 15 mL of a 1:1000 of alkaline phosphatase conjugated goat anti-rabbit IgG was added. After 2 hours incubation the blot was developed using FAST RED TR SALT (Sigma) and Naphthol-AS-MX phosphate (Sigma).

EXAMPLE 6

Bacterial Expression of Recombinant MIEP

Plasmid pUC19-MIEP containing the 1.3 kilobase pair MIEP gene insert, was digested with restriction endonucleases SpeI and EcoRI. The 1.1kbp fragment was isolated and purified on an agarose gel using standard techniques known in the art. Plasmid pTACSD, containing the two cistron TAC promoter and a unique ECORI site, was digested with ECORI. Blunt ends were formed on both the 1.3 kbp MIEP DNA and the pTACSD vector, using T4 DNA polymerase (Boehringer Mannheim) according to the manufacturer's directions. The blunt ended 1.3 kbp MIEP DNA was ligated into the blunt ended vector using T4 DNA ligase (Boehringer Mannheim) according to the manufacturer's directions. The ligated DNA was used to transform E. coli strain DH5aIQMAX (BRL) according to the manufacturer's directions. Transformed cells were plated onto agar plates containing 25 ug kantamycin/mL and 50 ug penicillin/mL, and incubated for about 15 hours at 37 C. A DNA oligonucleotide with a sequence homologous with MIEP was labelled with $^{32}P$ and used to screen nitrocellulose filters containing lysed denatured colonies from the plates of transformants using standard DNA hybridization techniques. Colonies which were positive by hybridization were mapped using restriction endonucleases to determine the orientation of the MIEP gene.

Expression of MIEP by the transformants was detected by Western blot analysis. Recombinant MIEP produced in the transformants comigrated on polyacrylamide gels with MIEP purified from OMPC vesicles, and was immunologically reactive with antibodies specific for MIEP.

EXAMPLE 7

Preparation of Purified MIEP from OMPC Vesicles or From Recombinant Cells by Polyacrylamide Gel Electrophoresis

Acrylamide/BIS (37.5:1) gels, 18 x 14 cm, 3 mm thick were used. The stacking gel was 4% polyacrylamide and the separating gel was 12% polyacrylamide. Approximately 5 µg of vesicle protein, or recombinant host cell protein, was used per gel. To 1 mL of OMPC vesicles was added 0.5 mL of sample buffer (4% glycerol, 300 mM DTT, 100 mM TRIS, 0.001% Bromophenol blue, pH 7.0). The mixture was heated to 105° C for 20 minutes and allowed to cool to room temperature before loading onto the gel. The gel was run at 200-400 milliamps, with cooling, until the Bromophenol blue reached the bottom of the gel. A verticle strip of the gel was cut out (about 1-2 cm wide) and stained with Coomassie/cupric acetate (0.1%). The strip was destained until the MIEP band (about 38 Kd) became visible. The strip was then placed into its original gel position and the MIEP area was excised from the remainder of the gel using a scalpel.

The excised area was cut into cubes (about 5 mm) and eluted with 0.01 M TRIS-buffer, pH 0.1. After 2 cycles of elution the eluate was evaluated for purity by SDS-PAGE. The eluate was combined with a common pool of eluates and dialysed against borate-buffer (0.1 M boric acid, pH 11.5). After dialysis the eluted protein was concentrated using an Amicon stirred cell with YM10 membranes (10,000 molecular weight cutoff). The material was further purified by passage through a PD10 sizing column (Pharmacia, Piscataway, NJ), and was stored at room temperature in borate buffer.

EXAMPLE 8

PREPARATION OF MIEP - cPND15 CONJUGATE:

To 10.5 mL of a MIEP solution (1.85 mg/mL, 19.4 mg total) contained in a 50 mL flask was added 2.6 mL of a 0.1 M, pH 11 borate buffer. The pH was adjusted to 10.8 with 5N NaOH after addition of 37 mg EDTA and 11 mg dithiothreitol. Then 346 mg of N-acetylhomocysteine thiolactone was added and the pH again adjusted to 11 with 5N NaOH. This solution was degassed, the air replaced with nitrogen and the solution aged for 23 hours under an atmosphere of nitrogen.

The sample was then dialized against 4L of pH9.5 borate containing 10 mL, EDTA for 7 hr; against a

fresh 4L for 22 hrs and finally against a pH 9.5 0.01M borate buffer containing 1.9 mg DTT for 16 hrs. This treatment afforded a solution that contained a total of 4.84 µmoles of thiol (by Ellman assay). This equates to 249 nanomoles SH/mg protein.

A 10 mg sample of maleimidated cPND15 from Example 13 was dissolved in 1 mL of $H_2O$ and 50 µL of this was used for a maleimide assay by the reverse Ellman method, to reveal 5.4 µmoles (total) of maleimide. A 0.9 mL (4.88 µmoles) aliquot of the solution was added to the thiolated MIEP solution (pH 9.5), which immediately became turbid and after 3 hrs and 40 minutes no thiol titer (by Ellman assay) remained.

The solution (14 mL) was dialyzed twice vs 4L of a pH 9.5, 0.01M borate buffer for 27.5 and 38 hrs respectively. An assay on 100 µL for amino acid composition gave the following results:

| | | |
|---|---|---|
| nanamoles/0.1 mL sample: | norleucine | 15.9 |
| | β-alanine | 13.7 |
| | lysine | 48.8 |

A Bradford protein assay on 100 µL showed 0.95 mg/mL. Using a molecular weight of 1111, this translates as 176.7 µg/mL of peptide. Thus the peptide to protein loading was 18.6%.

## EXAMPLE 9

### PREPARATION OF MIEP-cPND31 CONJUGATE:

To 6.5 mL of a MIEP solution (1.7 mg/mL) was added 1.5 mL of a pH 11, 0.1 M borate buffer and the pH adjusted to 11 with 5 µL of 5N NaOH. To this was added 21 mg of EDTA and 6.5 mg of DTT and solution was effected by tumbling for 15 min. Then 200 mg of N-acetylhomocysteine thiolactone was added, the solution degassed and the air replaced by $N_2$. After aging in the $N_2$ box for 1.5 hrs., the pH was adjusted to 10.66 with 5N NaOH, the degassing process repeated, and ageing continued for 20.5 hrs.

The solution was dialyzed vs 4L of 0.1M pH9.5 borate containing 0.01 M EDTA for 6.5 hr followed by 4L of 0.1M pH 9.6 borate, 10 mM EDTA containing 1 mg dithiolthreitol for 17 hr. An Ellman assay indicated 2.27 µmoles (total) of thiol which is equivalent to 205 nanomoles SH/mg protein.

To this thiolated protein solution was added 0.55 mL of maleimidated cPND31 from Example 14 (3.77 µmoles/mg, by reverse Ellman assay, 2.07 µmoles total). An instant turbidity was noted. An additional 0.5 mg of maleimidated cPND31 was added and the mixture was aged for 1 hour.

To remove unconjugated peptide, the mixture was dialyzed in dialysis tubing, having a molecular weight exclusion limit of 12,000-14,000, vs 4L of pH 9.48 0.1M borate for 5.25 hours and vs 4L of pH 9.68 0.01M borate for 66 hrs. A total of 8 mL of solution remained from which 200 µL was removed for amino acid analysis:

| | |
|---|---|
| norleucine | 22.8 nanomoles/200 µL . |
| lysine | 85.9 nanomoles/200 µL. |

The solution was then dialyzed vs 200 mL of pH 7.07 0.1 M phosphate buffer which was 5 M in urea, affording a final volume of 6.5 mL. A Bradford protein assay revealed 1.26 mg protein/mL (8.2 mg total). Thus, 0.912 µmoles peptide (8 mL X 22.8 nanomoles/0.2 mL) at a molecular weight of 1204 = 1.1 mg of peptide (total). Therefore, in this case, a peptide to protein loading of 13% was achieved.

## EXAMPLE 10

### Solid State Synthesis of Disulfide-Bonded cPND4:

A linear PND peptide was prepared on Wang resin using an ABI-431A peptide synthesizer, starting from Fmoc-L-Cys(Acm)-O-Wang resin (0.61 meq/gram). Fmoc chemistry and Fmoc-Amino Acid symmetrical anhydrides (4X excess, prepared in situ) were used as reagents on a 0.25 mmole scale to generate 745 mg of the peptide: (SEQ. ID. NO: 30)

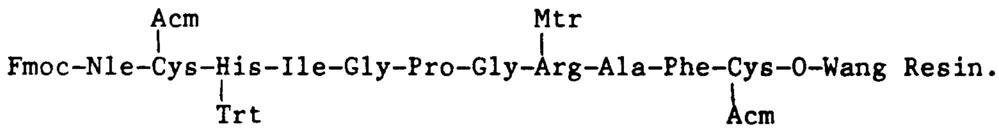

A solution of iodine in 5% methanol/anhydrous DMF (1 ml) was added to the dried, derivatized Wang resin shown above and stirred at room temperature for 4 hours. The resin was filtered, washed with anhydrous DMF (5 x 2 ml), and finally resuspended in DMF (2 ml). Two drops of a 0.1 M solution of sodium thiosulphate in water were added, and stirred for a few seconds. The resin was washed with aqueous 95% DMF (3 x 2 ml), anhydrous DMF (2 ml), methylene chloride (3 x 2 ml), ether (3 x 2 ml) and dried.

The Fmoc and other protecting groups were removed by treatment with 20% piperidine in DMF over 20 minutes, and the resin was washed and dried. The resin was cleaved from the disulfide bonded cyclic peptide by treatment with 95% TFA/4% ethane dithiol/1% thioanisole (1 ml) at room temperature for 6 hours. The solution was filtered, the resin washed with additional 100% TFA (3 x 1 ml), and the combined filtrate dried. Material that was insoluble in ether was removed by extraction (3 x 2 ml) and the solution redried.

Preparative HPLC using two 2.12 x 25 cm Vydac C18 reverse phase columns in series and a gradient elution of 20 to 24% $CH_3CN$ over 90' allowed isolation of a sharp peak eluting at 36.66' under these conditions. Analytical HPLC yielded a single peak upon co-chromatography of a known disulfide bonded cyclic standard with the product obtained from preparative HPLC. FAB-MS gave a $[M+H]^+$ of 1171, which is consistent with the the disulfide bonded cyclic structure cPND4: (SEQ. ID. NO: 30)

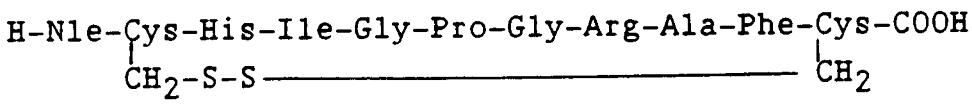

## EXAMPLE 11

### 1. Solution Synthesis of Peptide Bonded cPND15:

The linear peptide Cbz-Nle-Lys(Boc)-Gln-Arg(Mtr)-Gly-Pro-Gly-Arg(Mtr)-Ala -Phe was synthesized following solid-phase methods on an ABI 431A peptide synthesizer using 373 milligrams (0.1 mmoles) of commercially available Fmoc-Phenylalanyl-p- alkoxybenzyl alcohol resin. With the exception of norleucine, which was purchased in the benzyloxycarbonyl (Cbz) protected form, L-amino acids used were the fluorenylmethoxycarbonyl (Fmoc) derivatives having the appropriate acid-labile side chain protecting groups. The polypeptide-derivatized resin product was transferred to a sintered glass funnel, washed with dichloromethane, and dried, to yield 0.6 g of polypeptide-resin product.

The peptide was cleaved from the resin by treatment with 6 ml of a 95:2:3 mixture of TFA:1,2 ethanediol:anisole for 16 hours. The reaction mixture was filtered through a sintered glass funnel, the resin washed with 10 ml TFA, and the filtrates combined. Following concentration to about 1 to 2 ml of yellow oil, the linear peptide was recovered by trituration with 400 ml of diethyl ether, in 50 ml portions, and filtration on a sintered glass funnel. Dissolution with 100 ml 1% TFA followed by lyophilization yielded 298 mg of linear peptide.

The peptide powder was dissolved in 800 ml DMF, neutralized with 0.42 ml diisopropylethylamine, and treated with 0.077 ml diphenylphosphorylazide. The solution was stirred in the dark for 70 hours at 4°C to allow formation of the cyclic lactam. After quenching by addition of 3 ml glacial acetic acid, the reaction mixture was concentrated to about 1 to 2 ml of oil, dissolved in 10% aqueous acetic acid, and lyophilized.

The cyclic peptide was purified by G-15 size exclusion chromatography using 5% acetic acid as the mobile phase. Fractions, monitored by UV detection, containing the peptide were pooled and lyophilized to yield 135 mg of dry cyclic peptide. All results obtained were consistent with the structure cPND15: (SEQ. ID. NO: 31)

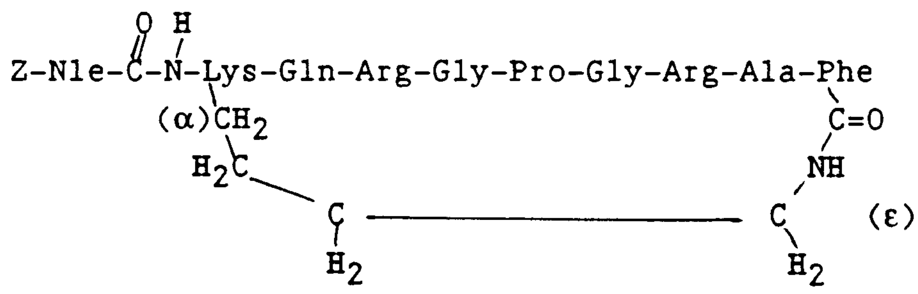

which may also be represented as: (SEQ. ID. NO: 31)

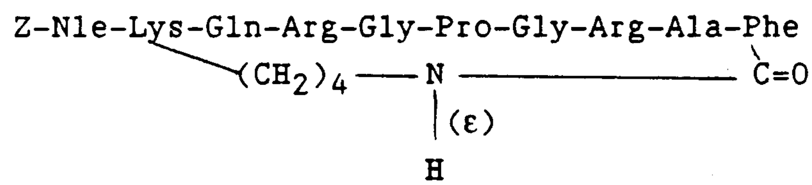

2. Deprotection of cPND15 to yield the hydrogen form:

Deprotection of cPND15 was achieved by dissolving the cyclic peptide in 20 ml of 30% aqueous acetic acid and hydrogenation at 40 psi for 16 hours over 100 mg of 10% palladium on carbon. The reaction mixture was filtered over celite to remove the catalyst, and the filtrate was lyophilized. Reverse phase HPLC using a Vydac C18 semi-prep column was utilized to obtain 8.5 mg of pure deprotected cyclic peptide. This method of deprotection is applicable to all peptides synthesized as the benzyloxycarbonyl N-protected peptide, to yield the free hydrogen form of the peptide which may now be activated at the amino terminus in preparation for conjugation. The structure of the product was confirmed by FAB-MS, analytical HPLC and amino acid analysis, and all results were consistent with the structure cPND15: (SEQ. ID. NO: 31)

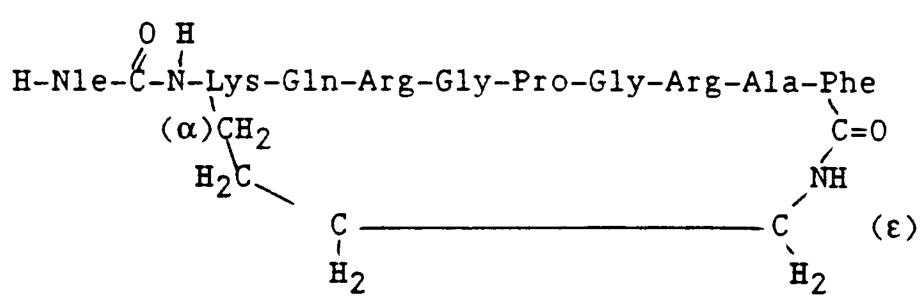

which may also be represented as: (SEQ. ID. NO: 31)

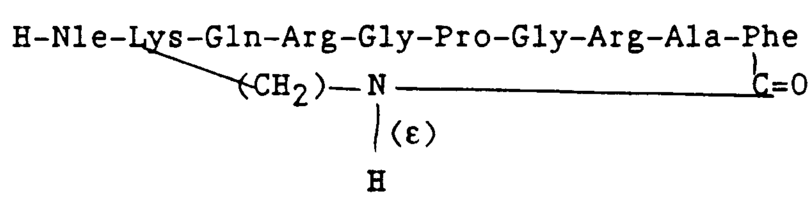

EXAMPLE 12

Synthesis of cPND31:

Two grams (0.6 meq/gram) of Fmoc-Phe-Wang resin was loaded on an ABI 431A synthesizer. Fmoc single coupling protocols were used to add Fmoc-Ala, Fmoc-Arg(Tos), Fmoc-Pro, Fmoc-Ile, Fmoc-His(Trt), Boc-Lys(Fmoc), and Cbz-Nle to produce 3.7 grams of linear peptide resin having the sequence:
Boc-Lys($N^{\epsilon}$-Z-Nle)-His(Trt)-Ile-Gly-Pro-Gly-Arg(Tos)-Ala-Phe.

The peptide was cleaved from the resin by treating with 95% TFA, 5% water for two hours. The resin was removed by filtration, the TFA removed from the filtrate by evaporation in vacuo, and the residue was triturated with diethyl ether. The precipitate was recovered by filtration and drying to yield 1.7 grams of linear peptide having the sequence:
H-Lys($N^{\epsilon}$-Z-Nle)-His-Ile-Gly-Pro-Gly-Arg(Tos)-Ala-Phe.

The peptide was treated with Boc-isoglutamine-ONp (0.71 grams, 2 nmoles,) and DIEA (0.35 ml, 2 mmoles) in DMF (10 ml) overnight at room temperature. The DMF was evaporated, and the residue treated with diethyl ether. The precipitate was recovered by filtration and washed with ethyl acetate. The dried peptide (1.9 grams) was treated with TFA (100 ml) for 0.5 hours. The TFA was evaporated in vacuo, the residue triturated with diethyl ether and the precipitate was recovered by filtration and dried.

The peptide was desalted on Sephadex G-10 in 10% aqueous acetic acid as the eluent. Peptide fractions were lyophilized to yield 1.2 grams (0.79 mmoles) of: (SEQ. ID. NO: 33)

H-isoGln-Lys($N^{\epsilon}$-Z-Nle)-His-Ile-Gly- Pro-Gly-
Arg(Tos)-Ala-Phe

Two batches (0.55 gm, 0.36 mmoles) of the peptide were separately dissolved in 1000 mL ice cold DMF and DIEA (0.16 mL, 0.9 mmoles) and DPPA (0.12 mL were added and the solutions were stirred overnight at room temperature. The DMF was evaporated in vacuo and the residues combined and solubilized in $CHCl_3$. The organic fraction was washed with 5% aqueous citric acid, then dried over $MgSo_4$ and evaporated to yield 0.78 gm of crude cyclic peptide. This material was treated with liquid HF (10 mL) containing anisole (1 mL) for two hours at 0°C. The HF was evaporated and the residue was purified by graidien elution on reveresed phase HPLC (Vydac C-18, 0-50% $CH_3CN$, over 50 minutes using 0.1 % aqueous TFA as the buffer) to give 250 mg of pure cPND31 (M+H=1204). (SEQ. ID. NO. 30)

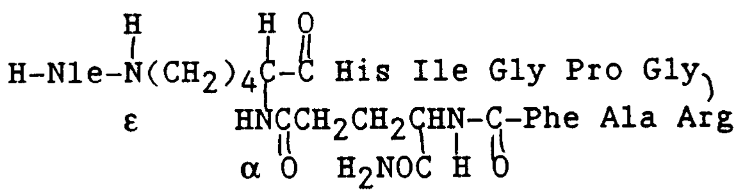

EXAMPLE 13

Preparation of MaleimidoPropionyl-cPND15:

10 milligrams of cPND15 trifluoroacetate salt was dissolved in 0.3 ml of a 1:2 mixture of $H_2O$:MeCN. The solution was cooled in an ice bath and then 100 µL of 0.345 M $NaHCO_3$ solution, followed by 3.5 mg of maleimidopropionic acid N-hydroxysuccinimide ester, was added. The reaction was allowed to proceed with stirring for one hour, followed by quenching with 3 µL of trifluroacetic acid. The reaction mixture was filtered through a 0.2 micron filter, and the filter was washed with 0.2 ml of water. The combined filtrates were injected onto a 2.15 X 25 cm Vydac C18 reverse phase column. The column was eluted isocratically for 10 minutes at a flow rate of 10 ml/min. with 25% MeCN/0.1% TFA, followed by gradient elution from 25 to 40% MeCN/0.1% TFA, over 20 minutes. The product eluting between 20 and 32 min was concentrated and lyophilized, yielding 7 mg of the trifluoroacetate salt of maleimidopropionyl-cPND15 as a white amorphous

powder. FAB-MS revealed a major ion (M + H) at 1262. Titration for maleimide by Ellman assay quenching gave a concentration of 0.54 μmoles per mg of the maleimidopropionyl-cPND15.

EXAMPLE 14

Preparation of Maleimidopropionyl-cPND31:

Following the procedure of Example 13, 37.6 mg of the trifluoroacetate salt of cPND31 was reacted with 8.3 mg of maleimidopropionyl N-hydroxysuccinimide ester in 0.4 ml of a 0.322 M $NaHCO_3$ solution and 1.2 ml of 1:2 $H_2O$:MeCN, followed by quenching with 10.5 μl of TFA. Preparative HPLC (30% MeCN/0.1% TFA isocratic for 10 minutes followed by gradient elution from 30-50% MeCN over 5 min gave a product peak eluting between 18-25 min. The lyophilized product weighed 26 mg, and the maleimide titer was 0.57 μM/mg. FAB-MS gave a major ion (M + H) at 1356. Amino acid analysis gave Nle = 460, β-alanine = 420 and Lys = 460 nmoles/mg.
NMR analysis gave a singlet at 6.93 ppm (maleimide H).

EXAMPLE 15

Protocol for Inoculation of Animals with the MIEP-cPND15 and MIEP-cPND31 conjugate of this Invention:

Alum was used as an adjuvant during the inoculation series. The inoculum was prepared by dissolving the conjugate in physiologic saline at a final conjugate concentration of 300 μg/ml. Preformed alum (aluminum hydroxide gel) was added to the solution to a final level of 500 μg/ml aluminum. The conjugate was allowed to adsorb onto the alum gel for two hours at room temperature. Following adsorption, the gel with the conjugate was washed twice with physiologic saline and resuspended in saline to a protein concentration of 300 μg/ml.

African green monkeys were individually inoculated with three 300 μg doses or three 100 μg doses of the conjugate adsorbed onto alum. Each dose was injected intramuscularly. The doses were delivered one month apart (week 0, 4, 8 and 28). The animals were bled at intervals of two weeks. Serum samples were prepared from each bleed to assay for the development of specific antibodies as described in the subsequent examples.

EXAMPLE 16

Analysis of Sera for Anti-Peptide IgG Antibodies:

Each serum sample is analyzed by enzyme-linked immunoadsorbent assay (ELISA). Polystyrene microtiter plates were coated with 0.5 μg per well of the synthetic peptide (not conjugated to MIEP) in phosphate-buffered physiological saline (PBS) at 4° C. Each well was then washed with PBS containing 0.05% TWEEN-20 (PBS-T). Test serum, diluted serially in PBS-T, was added to the peptide-containing wells and allowed to react with the adsorbed peptide for one hour at 36° C. After washing with PBS-T, alkaline phosphatase-conjugated goat anti-human IgG was added to the test wells and was allowed to react for one hour at 36° C. The wells were then washed extensively in PBS-T. Each well received 0.1% p-nitrophenyl phosphate in 10% diethanolamine, pH 9.8, containing 0.5 mM $MgCl_2 \bullet 6H_2O$. The ensuing reaction was allowed to proceed at room temperature for 30 minutes, at which time it was terminated by the addition of 3.0 N NaOH.

The greater the interaction of antibodies in the test serum with the peptide substrate, the greater is the amount of alkaline phosphatase bound onto the well. The phosphatase enzyme mediates the breakdown of p-nitrophenyl phosphate into a molecular substance which absorbs light at a wavelength of 405 nm. Hence, there exists a direct relationship between the absorbance at 405 nm of light at the end of the ELISA reaction and the amount of peptide-bound antibody.

All the monkeys inoculated with the maleimidopropionyl-cPND15-MIEP and malemidopropinyl cPND31-MIEP conjugates developed antibodies specifically capable of binding the peptide. Unconjugated, disulfide-bonded, cyclic peptide having identical primary sequence did not raise detectable anti-peptide antibodies when administered to African Green monkeys at 300 μg/dose at 0, 4, and 8 weeks.

EXAMPLE 17

Analysis of Sera for Activity which Specifically Neutralizes HIV Infectivity:

Virus-neutralizing activity is determined with an assay described by Robertson et al., J. Virol. Methods 20: 195-202 (1988). The assay measures specific HIV-neutralizing activity in test serum. The assay is based on the observation that MT-4 cells, a human T-lymphoid cell line, are readily susceptible to infection with HIV and, after a period of virus replication, are killed as a result of the infection.

The test serum is treated at 56°C for 60 minutes prior to the assay. This treatment is required to eliminate non-specific inhibitors of HIV replication. Heat treated serum, serially diluted in RPMI-1640 cell culture medium, is mixed with a standard infection dose of HIV. The dose is determined prior to the assay as containing the smallest quantity of virus required to kill all the MT-4 cells in the assay culture after a period of 7-8 days. The serum-virus mixture is allowed to interact for one hour at 37°C. It then is added to 1.0 x $10^5$ MT-4 cells suspended in RPMI-1640 growth medium supplemented with 10% fetal bovine serum. The cultures are incubated at 37°C in a 5% $CO_2$ atmosphere for 7 days.

At the end of the incubation period, a metabolic dye, DTT, is added to each culture. This dye is yellow in color upon visual inspection. In the presence of live cells, the dye is metabolically processed to a molecular species which yields a blue visual color. Neutralized HIV cannot replicate in the target MT-4 cells and therefore does not kill the cells. Hence, positive neutralization is assessed by the development of blue color following addition of the metabolic dye.

EXAMPLE 18

Preparation of a cyclic disulfide for conjugation:

1. PREPARATION OF cPND33:

H-Nle Cys Tyr Asn Lys Arg Lys Arg Ile His Ile Gly Pro Gly Arg Ala Phe Tyr Thr Thr Lys Asn Ile Ile Gly Cys-OH ($C_{135}H_{220}N_{42}O_{33}S_2$, formula weight = 3023.6) (SEQ.ID.NO: 22)

The 26mer was assembled on the Milligen # 9050 synthesizer, starting from partially racemised Fmoc-L-Cys(Trt)-OPKA resin (Milligen batch B 090426, 0.081 meq/g), using 2.47 g (0.200 meq). The theoretical yield is 604 mg. The resin was mixed with an equal volume of glass beads (Sigma 150-212 $\mu$m). The mixture completely filled two 1 x 10 cm columns, connected in series. Reagents were Fmoc-Pfp ester (except for threonine, which was dHBt), using four fold molar excess in N-methyl pyrrolidine solvent. Side chain protection was: Tyr (tert-butyl); Lys (Boc); Arg (Mtr); His (Boc); Thr (tert-butyl); Cys (Trt). The protocol was modified to give double coupling with $Lys^7$; $Ile^9$: $Ile^{11}$; $Gly^{12}$; $Pro^{13}$; $Gly^{14}$; $Arg^{15}$; $Phe^{17}$; $Tyr^{18}$; $Thr^{19}$; $Thr^{20}$; $Ile^{23}$; $Ile^{24}$. Acylation recycle times were extended from 30 to 60 mminutes for all units, except for $Gly^{14}$ and $Ala^{16}$, and to 90 minutes for $Ile^9$ (2x); $Ile^{11}$ (2x); $Ile^{23}$ (2x) and $Ile^{24}$ (2x). The derivatized resin was maintained as the free terminal amine which was washed with $CH_2Cl_2$ and air-dried.

The mixture of dry derivatized resin and glass beads was resuspended in 95% TFA, 4% ethane dithiol, 1% $CH_3SPh$ (30 mL) at 23°C in a sealed flask, with gentle stirring on an oscillating tray for 8 hours. The bright yellow mixture was then filtered and the insolubles were thoroughly extracted with 100% TFA (3 x 20 mL). The combined dark orange filtrates were evaporated to give a pale tan, oily gum. On trituration with ether (20 mL) this material instantly became a colorless solid, which was transferred to a filter by triturating with additional ether (3 x 20 mL). After drying, the crude product was obtained as a fine colorless powder (583 mg).

Analytical reverse phase HPLC [aqueous 0.1% TFA/22% $CH_3CN$, $\lambda$ = 215 nm, A = 0.05, 2.0 mL/min.] on a 0.46 x 25.0 cm Vydac $C_{18}$ column of about a 50 $\mu$g sample, dissolved in 50 $\mu$L aqueous 0.1 % TFA/20% $CH_3CN$, 4 $\mu$L injected, revealed a major component (36.29') and a later eluting minor component. These were separately collected after injection of a 30 mg and another 50 mg aliquot of the sample onto two 2.21 x 25.0 cm preparative Vydac $C_{18}$ columns in series [linear gradient over 60': 0.1% TFA/23-27% $CH_3CN$, $\lambda$ = 215 nm, A = 3.00, 10 mL/min]. A total of 35.2 mg of the earlier eluting material (44.45') and

8.2 mg of the later eluting material was recovered following lyophilization. FAB-MS of the major product gave a $[M+H]^+ = 3022.1$ and an $[M+Na]^+ = 3044.2$, which is consistent with the calculated mass.

2. PREPARATION OF THE CYCLIC DISULFIDE:

```
H-Nle Cys Tyr Asn Lys Arg Lys Arg Ile His Ile Gly Pro
       |        Gly Arg Ala Phe Tyr Thr Thr Lys Asn
       |        Ile Ile Gly Cys-OH
       S ------------------S
```

(SEQ. ID. NO: 22)

a. $K_3Fe(CN)_6$ INDUCED OXIDATION:

The linear 26 mer dithiol compound (35.0 mg) was dissolved in degassed distilled water (38 mL) at 23 °C to give a clear colorless solution at pH 2.73. The pH was adjusted to 8.5 with 0.1 N $NH_4OH$, and the solution was covered with an atmosphere of nitrogen. An aliquot of the material was immediately run on analytical reverse phase HPLC and found to be undergoing oxidation as evidenced by the appearance of an early peak.

With magnetic stirring, a freshly prepared solution of 0.01 M $K_3Fe(CN)_6$ was added by power driven hypodermic syringe at 23° C under nitrogen. Analysis of a small aliquot by HPLC revealed total conversion of starting material to an earlier elution time. The reaction mixture (pH 8.3) was mixed with 10% aqueous acetic acid and stirred to give a pH of 4.0. The solution was filtered to remove insoluble material, and the faintly yellow solution was evaporated and then lyophilized to give about 27.9 mg of a pale yellow powder. The material was dissolved in 0.1% TFA/20% $CH_3CN$ and gradient eluted on a preparative HPLC. A major early eluting peak and a later eluting peak (4:1) were separately collected and lyophilized to yield 6.1 mg of the early and 1.5 mg of the late eluting material. FAB-MS analysis of the early eluting material: $[M+H]^+$ 3019.7; $[M+Na]^+$ 3042.5; FAB-MS analysis of the late eluting material: $[M+H]^+$ 3020.0; $[M+Na]^+$ early material = 3041.5; all of which corresponds to the correct mass for the cyclized cPND33. The later eluting material is the D-cysteine carboxy terminus diastereomer.

Amino acid analysis of the products gave the predicted amino acid compositions for the cyclized products and confirmed that the later eluting material is the D-cysteine containing diastereomer.

b. AIR OXIDATION:

The linear 26 mer prepared in (1) above (86 mg, 28.4 µmoles) was dissolved in aqueous 0.1% TFA/20% acetonitrile (284 mL) at 23° C and the solution was allowed to stand open to the air. Cyclization was monitored by reverse phase HPLC and the sample was found to be almost completely converted to the early eluting material, with almost complete dissappearance of starting linear material, by t = 24 hours. The clear, colorless solution was evaporated to about 8 mL at which point an additional 10 mg sample prepared in the same way as the 86 mg, was added. The combined sample was evaporated to about 9 mL. The cloudy colorless solution was subjected to HPLC separation, in two separate runs, on two 2.12 x 25.0 cm Vydac $C_{18}$ columns in series. Two fractions were separately collected, an early eluting peak and a later eluting peak. Each peak was separately evaporated and lyophilized to yield 30.1 mg and 9.7 mg of the early and late materials respectively. The early eluting material was combined with other preparations of early eluting cyclized material to yield a total of 47.5 mg of a faintly bluish fluffy powder. Analytical HPLC of this material gave a single peak.

3. PREPARATION OF 3-MALEIMIDOPROPIONIC ACID ANHYDRIDE

3-Maleimidopropionic acid (226 mg) was covered with 5 mL of acetic anhydride and the mixture was heated at 130° C for 3.75 hr, and then aged over night at room temperatue. The solution was concentrated to an oil and the NMR spectrum ($CDCl_3$) indicated a mixture of the homoanhydride and the mixed anhydride of acetic and maleimidopropionic acids. The starting acid shows the methylene adjacent to the

carbonyl as a triplet centered at 2.68 ppm whereas in the anhydride these resonances appear at 2.81 ppm. Purification was effected by fractional sublimation, first at 70°C and 0.2 mm and then at 120°C and 0.2 mm. The latter fraction was removed from the apparatus by dissolving in $CDCl_3$, affording 34 mg of pure homoanhydride on evaporation of the solvent. This was recrystallized from $CDCl_3$ and cyclohexane affording material melting at 143-147°C.
Calcd. for $C_{14}H_{12}N_2O_7$: C,52,51;H,3.78;N,8.75.
Found: C,51.73;H3.67;N,8.16. 200 MHz NMR
($CDCl_3$):2.83 (2H,t)3.84 (2H,t),6.73 (2H,s).

4. "SELECTIVE" ACYLATION OF cPND33

cPND33 (22.5 mg; at estimated 70% peptide is equivalent to 15.75 mg or 5.212 micromoles) was dissolved in 12.0 mL of a 0.1M pH 5.25 morpholinoethane sulfonic acid buffer and cooled in an ice bath. Analysis of this solution and progress of the reaction was followed by HPLC on a 25 cm ODS column using 25% aqueous acetonitrile: 0.1% trifluoroacetic acid (TFA) as eluent. Maleimidopropionic acid anhydride (2.0 mg, 6.25 micromoles) was dissolved in 0.600 mL of dry tetrahydrofuran, and 0.5 mL of this solution (corresponding to 5.2 micromoles of anhydride) was added to the above peptide solution. After 30 sec., a 7 microliter aliquot was removed and evaluated by HPLC. This assay was repeated at 0.25, 0.50, 1.25, 2.25 and 3.0 hr. After 3.5 hr the solution was lyophilized. The lyophylizate was dissolved in 2.0 mL of 20% aqueous acetonitrile, filtered through a 0.2 micron filter and preparatively chromatographed in three 0.700 mL runs on a 21.2 mm x 25 cm Zorbax C-18 column. The following elution program was used: flow rate = 10 mL/min; isocratic elution with 25% aqueous acetonitrile/0.1% TFA (12 min); gradient to 28% acetonitrile (10 min); gradient to 35% acetonitrile (8 min). The tail fractions were isolated by concentration and lyophilization to afford 8.9 mg of recovered starting material (penultimate fraction) and 9.6 mg of a product which had a mass spectrum (FAB) indicatiing a molecular weight of 3172 (i.e the mono-maleimidopropionyl derivative of cPND33).

The product was further characterized by a sequence analysis looking for the absence of lysine (the absence of any sequence would imply terminal amino acylation). The results indicate that most but not all of the maleimidopropionyl moiety is bonded to the lysine closest to the carboxy terminus.

## EXAMPLE 19

### Mitogenic Activity of MIEP

MIEP purified from N. meningitidis OMPC was tested for mitogenic activity in a lymphocyte proliferation assay. Murine splenic lymphocytes were obtained from C3H/HeN, C3H/FeJ, C3H/HeJ, or Balb/c mice. The mice were either naive or had previously been vaccinated with PRP-OMPC. The spleen cells were passed through a sterile, fine mesh screen to remove the stromal debris, and suspended in K medium [RPMI 1640 (GIBCO) plus 10% fetal calf serum (Armour), 2 mM Glutamine (GIBCO), 10 mM Hepes (GIBCO), 100 u/mL penicillin/100/µg/mL streptomycin (GIBCO), and 50 µM β-mercaptoethanol (Biorad)]. Following pipetting to disrupt clumps of cells, the suspension was centrifuged at 300 x g for 5 minutes, and the pellet was resuspended in red blood cell lysis buffer [90% 0.16 M $NH_4Cl$ (Fisher), 10% 0.7 TRIS-HCl (Sigma), pH 7.2] at room temperature, 0.1 mL cells/mL buffer for two minutes. Cells were underlayered with 5 mL of fetal calf serum and centrifuged at 4,000 x g for 10 minutes, then washed with K medium two times and resuspended in K medium at $5 \times 10^6$ cells/mL. These cells were plated (100 µL/well) into 96 well plates along with 100 µL of protein or peptide sample, in triplicate.

The MIEP of N. meningitidis was purified as previously described in Example 7. Control proteins included bovine serum albumin, PRP-OMPC and OMPC itself, and lipopolysaccharide (endotoxin). All samples were diluted in K medium to concentrations of 1, 6.5, 13, 26, 52, 105, and 130 µg/mL, then plated as described above such that their final concentrations were one-half of their original concentrations. Triplicate wells were also incubated for each type of cell suspended in K medium only, to determine the baseline of cell proliferation.

On day 3, 5, or 7 in culture, the wells were pulsed with 25 µL of $^3$H-thymidine (Amersham) containing 1 mCi/25 µL. The wells were harvested 16-18 hours later on a Skatron harvester, and counts per minute (CPM) was measured in a liquid scintillation counter. The net change in cpm was calculated by subtracting the mean number of cpm taken up per well by cells in K medium alone, from the mean of the experimental cpm. The stimulation index was determined by dividing the mean experimental cpm by the mean cpm of the control wells.

As shown in Figure 2, MIEP as well as OMPC and PRP-OMPC vaccine resulted in proliferation of lymphocytes from previously vaccinated mice. This mitogenic activity did not appear to be due to lipopolysaccharide (LPS) since the MIEP was free of detectable LPS, measured by rabbit pyrogenicity assays, and the proliferative effect was greater than that which could have been caused by LPS present in amounts below the level of detectability on silver stained polyacrylamide gels.

While the foregoing specification teaches the principles of the present invention, with examples provided for the purpose of illustration, it will be understood that the practice of the invention encompasses all the usual variations, adaptations, modifications, or deletions as come within the scope of the following claims and its equivalents.

SEQUENCE LISTING

(2) INFORMATION FOR SEQ ID NO:1:

(i) SEQUENCE CHARACTERISTICS:

(A) LENGTH: 41 amino acids

(B) TYPE: amino acid

(D) TOPOLOGY: linear

(ii) MOLECULE TYPE: peptide

(iii) HYPOTHETICAL: NO

(v) FRAGMENT TYPE: internal

(ix) FEATURE:

(A) NAME/KEY: Disulfide-bond

(B) LOCATION: 3..38

(xi) SEQUENCE DESCRIPTION: SEQ ID NO:1:

Ile Asn Cys Thr Arg Pro Asn Asn Asn Thr Arg Lys Ser Ile Arg Ile
1 5 10 15

Gln Arg Gly Pro Gly Arg Ala Phe Val Thr Ile Gly Lys Ile Gly Asn
20 25 30

Met Arg Gln Ala His Cys Asn Ile Ser
35 40

(2) INFORMATION FOR SEQ ID NO:2:

(i) SEQUENCE CHARACTERISTICS:

(A) LENGTH: 24 amino acids

(B) TYPE: amino acid

(D) TOPOLOGY: linear

(ii) MOLECULE TYPE: peptide

(xi) SEQUENCE DESCRIPTION: SEQ ID NO:2:

Tyr Asn Lys Arg Lys Arg Ile His Ile Gly Pro Gly Arg Ala Phe Tyr
1 5 10 15

Thr Thr Lys Asn Ile Ile Gly Thr
20

(2) INFORMATION FOR SEQ ID NO:3:

(i) SEQUENCE CHARACTERISTICS:

(A) LENGTH: 25 amino acids

(B) TYPE: amino acid

(D) TOPOLOGY: linear

(ii) MOLECULE TYPE: peptide

(xi) SEQUENCE DESCRIPTION: SEQ ID NO:3:

```
Asn Asn Thr Thr Arg Ser Ile His Ile Gly Pro Gly Arg Ala Phe Tyr
1               5                   10                  15

Ala Thr Gly Asp Ile Ile Gly Asp Ile
            20                  25
```

(2) INFORMATION FOR SEQ ID NO:4:

(i) SEQUENCE CHARACTERISTICS:

(A) LENGTH: 25 amino acids

(B) TYPE: amino acid

(D) TOPOLOGY: linear

(ii) MOLECULE TYPE: peptide

(xi) SEQUENCE DESCRIPTION: SEQ ID NO:4:

Asn Asn Thr Arg Lys Ser Ile Arg Ile Gln Arg Gly Pro Gly Arg Ala
1 5 10 15

Phe Val Thr Ile Gly Lys Ile Gly Asn
20 25

(2) INFORMATION FOR SEQ ID NO:5:

(i) SEQUENCE CHARACTERISTICS:
(A) LENGTH: 18 amino acids
(B) TYPE: amino acid
(D) TOPOLOGY: linear

(ii) MOLECULE TYPE: peptide

(xi) SEQUENCE DESCRIPTION: SEQ ID NO:5:

Arg Ile Gln Arg Gly Pro Gly Arg Ala Phe Val Thr Ile Gly Lys Ile
1 5 10 15

Gly Asn

(2) INFORMATION FOR SEQ ID NO:6:

(i) SEQUENCE CHARACTERISTICS:
(A) LENGTH: 11 amino acids
(B) TYPE: amino acid
(D) TOPOLOGY: linear

(ii) MOLECULE TYPE: peptide

(xi) SEQUENCE DESCRIPTION: SEQ ID NO:6:

Arg Ile Gln Arg Gly Pro Gly Arg Phe Val Thr
1 5 10

(2) INFORMATION FOR SEQ ID NO:7:

(i) SEQUENCE CHARACTERISTICS:
(A) LENGTH: 8 amino acids
(B) TYPE: amino acid
(D) TOPOLOGY: linear

(ii) MOLECULE TYPE: peptide

(xi) SEQUENCE DESCRIPTION: SEQ ID NO:7:

His Ile Gly Pro Gly Arg Ala Phe
1 5

(2) INFORMATION FOR SEQ ID NO:8:

(i) SEQUENCE CHARACTERISTICS:
(A) LENGTH: 6 amino acids
(B) TYPE: amino acid
(D) TOPOLOGY: linear

(ii) MOLECULE TYPE: peptide

(xi) SEQUENCE DESCRIPTION: SEQ ID NO:8:

Gly Pro Gly Arg Ala Phe
1 5

(2) INFORMATION FOR SEQ ID NO:9:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:9:

Ile Gln Arg Gly Pro Gly Arg Ala Phe
1 5

(2) INFORMATION FOR SEQ ID NO:10:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:10:

Ile Tyr Ile Gly Pro Gly Arg Ala Phe
1 5

(2) INFORMATION FOR SEQ ID NO:11:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:11:

Ile Ala Ile Gly Pro Gly Arg Thr Leu
1 5

(2) INFORMATION FOR SEQ ID NO:12:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:12:

Val Thr Leu Gly Pro Gly Arg Val Trp
1 5

(2) INFORMATION FOR SEQ ID NO:13:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:13:

Ile Thr Lys Gly Pro Gly Arg Val Ile
1 5

(2) INFORMATION FOR SEQ ID NO:14:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:14:

Thr Pro Ile Gly Leu Gly Gln Ser Leu
1 5

(2) INFORMATION FOR SEQ ID NO:15:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:15:

Thr Pro Ile Gly Leu Gly Gln Ala Leu
1 5

(2) INFORMATION FOR SEQ ID NO:16:

(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:16:

Ile His Phe Gly Pro Gly Gln Ala Leu
1 5

(2) INFORMATION FOR SEQ ID NO:17:
(i) SEQUENCE CHARACTERISTICS:
(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:17:

Ile Arg Ile Gly Pro Gly Lys Val Phe
1 5

(2) INFORMATION FOR SEQ ID NO:18:

(i) SEQUENCE CHARACTERISTICS:
(A) LENGTH: 4 amino acids
(B) TYPE: amino acid
(D) TOPOLOGY: both

(ii) MOLECULE TYPE: peptide

(xi) SEQUENCE DESCRIPTION: SEQ ID NO:18:

Gly Pro Gly Arg
1

(2) INFORMATION FOR SEQ ID NO:19:

(i) SEQUENCE CHARACTERISTICS:
(A) LENGTH: 4 amino acids
(B) TYPE: amino acid
(D) TOPOLOGY: both

(ii) MOLECULE TYPE: peptide

(xi) SEQUENCE DESCRIPTION: SEQ ID NO:19:

Gly Pro Gly Lys
1

(2) INFORMATION FOR SEQ ID NO:20:

(i) SEQUENCE CHARACTERISTICS:
(A) LENGTH: 4 amino acids
(B) TYPE: amino acid
(D) TOPOLOGY: both

(ii) MOLECULE TYPE: peptide

(xi) SEQUENCE DESCRIPTION: SEQ ID NO:20:

Gly Pro Gly Gln
1

(2) INFORMATION FOR SEQ ID NO:21:

(i) SEQUENCE CHARACTERISTICS:
(A) LENGTH: 4 amino acids
(B) TYPE: amino acid
(D) TOPOLOGY: both

(ii) MOLECULE TYPE: peptide

(xi) SEQUENCE DESCRIPTION: SEQ ID NO:21:

Gly Leu Gly Gln
1

(2) INFORMATION FOR SEQ ID NO:22:

(i) SEQUENCE CHARACTERISTICS:
(A) LENGTH: 26 amino acids
(B) TYPE: amino acid
(D) TOPOLOGY: both

(ii) MOLECULE TYPE: peptide

(ix) FEATURE:
(A) NAME/KEY: Modified-site
(B) LOCATION: 1
(D) OTHER INFORMATION: /label= Nle
/note= "norleucine"

(ix) FEATURE:
(A) NAME/KEY: Disulfide-bond
(B) LOCATION: 2..26

(xi) SEQUENCE DESCRIPTION: SEQ ID NO:22:

Leu Cys Tyr Asn Lys Arg Lys Arg Ile His Ile Gly Pro Gly Arg Ala
1 5 10 15

Phe Tyr Thr Thr Lys Asn Ile Ile Gly Cys
20 25

(2) INFORMATION FOR SEQ ID NO:23:

(i) SEQUENCE CHARACTERISTICS:
(A) LENGTH: 8 amino acids
(B) TYPE: amino acid
(D) TOPOLOGY: both

(ii) MOLECULE TYPE: peptide

(xi) SEQUENCE DESCRIPTION: SEQ ID NO:23:

```
Gln Arg Gly Pro Gly Arg Ala Phe
1               5
```

(2) INFORMATION FOR SEQ ID NO:24:

(i) SEQUENCE CHARACTERISTICS:
(A) LENGTH: 25 base pairs
(B) TYPE: nucleic acid
(C) STRANDEDNESS: both
(D) TOPOLOGY: linear

(ii) MOLECULE TYPE: cDNA

(xi) SEQUENCE DESCRIPTION: SEQ ID NO:24:

ACTAGTTGCA ATGAAAAAAT CCCTG 25

(2) INFORMATION FOR SEQ ID NO:25:

(i) SEQUENCE CHARACTERISTICS:
(A) LENGTH: 22 base pairs
(B) TYPE: nucleic acid
(C) STRANDEDNESS: both
(D) TOPOLOGY: linear

(ii) MOLECULE TYPE: cDNA

(xi) SEQUENCE DESCRIPTION: SEQ ID NO:25:

GAATTCAGAT TAGGAATTTG TT 22

(2) INFORMATION FOR SEQ ID NO:26:

(i) SEQUENCE CHARACTERISTICS:
(A) LENGTH: 12 base pairs
(B) TYPE: nucleic acid
(C) STRANDEDNESS: both
(D) TOPOLOGY: linear

(ii) MOLECULE TYPE: cDNA

(xi) SEQUENCE DESCRIPTION: SEQ ID NO:26:

AGCTCGGATC CG 12

(2) INFORMATION FOR SEQ ID NO:27:

(i) SEQUENCE CHARACTERISTICS:
(A) LENGTH: 23 base pairs
(B) TYPE: nucleic acid
(C) STRANDEDNESS: both
(D) TOPOLOGY: linear

(ii) MOLECULE TYPE: cDNA

(xi) SEQUENCE DESCRIPTION: SEQ ID NO:27:

AAGCTCGGAT CCTAGTTGCA ATG 23

(2) INFORMATION FOR SEQ ID NO:28:

(i) SEQUENCE CHARACTERISTICS:
(A) LENGTH: 41 base pairs
(B) TYPE: nucleic acid
(C) STRANDEDNESS: both
(D) TOPOLOGY: linear

(ii) MOLECULE TYPE: cDNA

(xi) SEQUENCE DESCRIPTION: SEQ ID NO:28:

CTAAGCTTAA CAAAATGGAC GTTACCTTGT ACGGTACAAT T 41

(2) INFORMATION FOR SEQ ID NO:29:

(i) SEQUENCE CHARACTERISTICS:
(A) LENGTH: 24 base pairs
(B) TYPE: nucleic acid
(C) STRANDEDNESS: both
(D) TOPOLOGY: linear

(ii) MOLECULE TYPE: cDNA

(xi) SEQUENCE DESCRIPTION: SEQ ID NO:29:

ACGGTACCGA AGCCGGGTTT CAAG 24

(2) INFORMATION FOR SEQ ID NO:30:

(i) SEQUENCE CHARACTERISTICS:
(A) LENGTH: 11 amino acids
(B) TYPE: amino acid
(D) TOPOLOGY: both

(ii) MOLECULE TYPE: peptide

(ix) FEATURE:
(A) NAME/KEY: Modified-site
(B) LOCATION: 1
(D) OTHER INFORMATION: /label= Nle
/note= "norleucine"

(xi) SEQUENCE DESCRIPTION: SEQ ID NO:30:

```
Leu Cys His Ile Gly Pro Gly Arg Ala Phe Cys
1               5                   10
```

(2) INFORMATION FOR SEQ ID NO:31:

(i) SEQUENCE CHARACTERISTICS:
(A) LENGTH: 10 amino acids
(B) TYPE: amino acid
(D) TOPOLOGY: both

(ii) MOLECULE TYPE: peptide

(ix) FEATURE:
(A) NAME/KEY: Modified-site
(B) LOCATION: 1
(D) OTHER INFORMATION: /label= Nle
/note= "norleucine"

(xi) SEQUENCE DESCRIPTION: SEQ ID NO:31:

```
Leu Lys Gln Arg Gly Pro Gly Arg Ala Phe
1               5                   10
```

(2) INFORMATION FOR SEQ ID NO:32:

(i) SEQUENCE CHARACTERISTICS:
(A) LENGTH: 4 amino acids
(B) TYPE: amino acid
(D) TOPOLOGY: both

(ii) MOLECULE TYPE: peptide

(xi) SEQUENCE DESCRIPTION: SEQ ID NO:32:

```
Gly Pro Gly Val
1
```

(2) INFORMATION FOR SEQ ID NO:33:

(i) SEQUENCE CHARACTERISTICS:
(A) LENGTH: 11 amino acids
(B) TYPE: amino acid
(D) TOPOLOGY: both

(ii) MOLECULE TYPE: peptide

(ix) FEATURE:
(A) NAME/KEY: Modified-site
(B) LOCATION: 3
(D) OTHER INFORMATION: /label= Nle
/note= "norleucine"

(ix) FEATURE:
(A) NAME/KEY: Modified-site
(B) LOCATION: 1
(D) OTHER INFORMATION: /label= iGln
/note= "isoglutamine"

(xi) SEQUENCE DESCRIPTION: SEQ ID NO:33:

```
Gln Lys Leu His Ile Gly Pro Gly Arg Ala Phe
1               5                   10
```

## Claims

1. A conjugate comprising the major immune enhancing protein, MIEP, covalently linked to peptides comprising Human Immunodeficiency Virus (HIV) Principle Neutralizing Determinants (PNDs), or peptides immunologically equivalent therewith.

2. The conjugate of Claim 1 wherein the immunogenic protein consists essentially of MIEP purified from the outer membrane protein complex (OMPC) of Neisseria meningitidis b and the PND peptides have linear structures, disulfide bonded cyclic structures, amide bonded cyclic structures, or thioether bonded cyclic structures.

3. The conjugate of Claim 2 wherein the PND peptides comprise the sequence -GlyProGlyArg-, (SEQ. ID.

NO: 18) -GlyProGlyLys-, (SEQ. ID. NO: 19) -GlyProGlyVal-, (SEQ. ID. NO: 32) -GlyProGlyGln-, (SEQ. ID. NO: 20) -GlyLeuGlyGln-, (SEQ. ID. NO: 21) -GlyProGlyArgAlaPhe-, (SEQ. ID. NO: 8) -HisIleGlyProGlyArgAlaPhe-, (SEQ. ID. NO: 7) or -GlnArgGlyProGlyArgAlaPhe- (SEQ. ID. NO: 23).

**4.** The conjugate of Claim 3 wherein the PND peptides have a core or loop amino acid structure:

$-x-X_n-X_1-X_2$-Gly-Pro-Gly-Arg-$X_3-X_4-X_m-y-$

or a pharmaceutically acceptable salt thereof, wherein:

$X_1$ is:
a) serine,
b) proline,
c) arginine,
d) histidine, or
e) glutamine;

$X_2$ is:
a) isoleucine,
b) arginine,
c) valine, or
d) methionine;

$X_n$ is either a bond or a peptide of up to 15 amino acids;

$X_3$ is:
a) alanine,
b) arginine, or
c) valine;

$X_4$ is:
a) phenylalanine,
b) isoleucine,
c) valine, or
d) leucine;

$X_m$ is bond or a peptide of up to 15 amino acids; and

-x- and -y- are joined to each other through a covalent structure, to form a cyclic peptide, which is covalently bound to MIEP.

**5.** The conjugate of Claim 4 wherein the cyclic covalent structure between x and y is comprised of:
a) an amide bond between an amino group on one side of the loop amino acids and a carboxyl group on the other side of the loop amino
acids, b) a xylylene thioether or lower alkyl thioether bond between sulfhydryl containing amino acids on either side of the loop amino acids, or
c) a disulfide bond between sulfhydryl containing amino acids on either side of the loop amino acids.

**6.** The conjugate of Claim 5 having the structure:

$_j$(PEP-A-)-MIEP

or a pharmaceutically acceptable salt thereof wherein:

PEP consists essentially of an HIV PND;

j is the peptide loading and is between 1% and 50% of the total conjugate protein;

-A- consists essentially of the linkage:

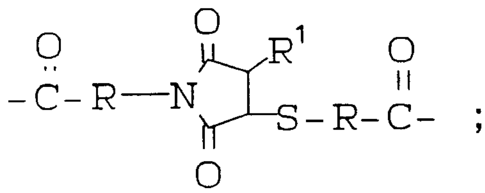

wherein:

- -S- is sulfur;
- -R- is:
  - a) -lower alkylene,
  - b) -substituted lower alkylene,
  - c) -cycloalkylene,
  - d) -substituted cycloalkylene,
  - e) -phenylene; and
- $-R^1$ is:
  - a) hydrogen, or
  - b) $-SO_3H$.

**7.** The conjugate of Claim 6 wherein the PEP is limited to:

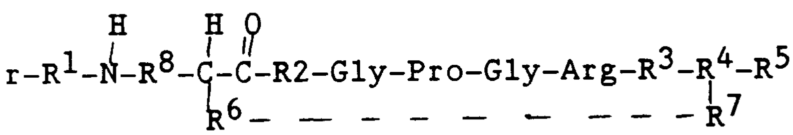

or a pharmaceutically acceptable salt thereof, wherein:

- r is the position of linkage between PEP and MIEP;
- $R^1$ is:
  - a) a bond, or
  - b) an amino acid or a peptide of 2 to 5 amino acids, optionally including a marker amino acid;
- $R^2$ is :
  - a) either a bond, an amino acid, or a peptide of 2 up to 17 amino acids, or
  - b) a peptide of between 2 to 17 amino acids,;
- $R^3$ is:
  - a) either a bond, an amino acid, or a peptide of 2 up to 17 amino acids, or
  - b) a peptide of between 2 to 17 amino acids;

$R^2$ and $R^3$ cannot both be option (a);

- $R^4$ is:
  - a) -NH-CH-CO- with $R^7$ bonded to the methine carbon if $R^7$ is $R^8$, or
  - b) a bond from $R^3$ to $R^7$ and $R^5$

  if $R^7$ is:

$-C=O$, or 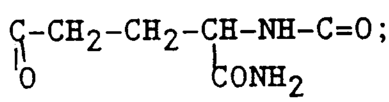

- $R^5$ is:
  - a) an amino acid or a peptide of two to five amino acids, optionally including a marker amino acid,
  - b) -OH,
  - c) -COOH,

d) $-CONH_2$,
e) $-NH_2$, or
f) -absent;

$R^6$ is:
a) an amino acid side chain, selected from the side chain of any of the common L or D amino acids, (see table of Definitios and Abbreviations), if the optional bond (--------) to $R^7$ is absent,
b) $-R^8$-S-S-, or $-R^8-S-R^8-R^9-R^8$-S-, if $R^7$ is $R^8$, or
c) $-R^8$-NH- if $R^7$ is

$-C=O$, or 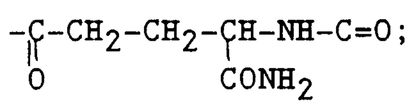

$R^7$ is:
a) $-R^8$-,
b) $-C=O$, or
c)

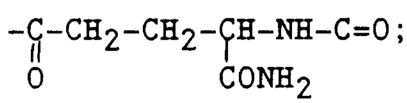

$R^8$ is a lower alkyl of between zero and eight carbons;
$R^9$ is:
a) lower alkylene, or
b) xylylene; and
$-R^2$-Gly-Pro-Gly-Arg-$R^3$- is the PEP core or loop amino acid structure.

**8.** The conjugate of Claim 7 wherein the PEP core amino acid structure $-R^2$-Gly-Pro-Gly-Arg-$R^3$-is:

$-X_nX_1X_2$-Gly-Pro-Gly-Arg-$X_3X_4X_m$-

wherein:
$X_1$ is a constituent of $R^2$ selected from:
a) serine,
b) proline,
c) arginine,
d) histidine, and
e) glutamine;
$X_2$ is a constituent of $R^2$ selected from:
a) isoleucine,
b) arginine,
c) valine, and
d) methionine;
$X_n$ is a constituent of $R^2$ and is either a bond, an amino acid, or a peptide of 2 up to 15 amino acids;
$X_3$ is a constituent of $R^3$ selected from:
a) alanine,
b) arginine, and
c) valine;
$X_4$ is a constituent of $R^3$ and is selected from:
a) phenylalanine,
b) isoleucine,

c) valine, and
d) leucine; and

$X_m$ is a constituent of $R^3$ and is a bond, an amino acid, or a peptide of 2 up to 15 amino acids.

**9.** The conjugate of Claim 8 wherein PEP is comprised of one or more of the peptides having the structure:

| | Peptide Sequence | Name | SEQ ID NO: |
|---|---|---|---|
| a) | r-Tyr Asn Lys Arg Lys Arg Ile His Ile Gly Pro Gly Arg Ala Phe Tyr Thr Thr Lys Asn Ile Ile Gly Thr | PND142 | 2 |
| b) | r-Asn Asn Thr Thr Arg Ser Ile His Ile Gly Pro Gly Arg Ala PheTyr Ala Thr Gly Asp Ile Ile Gly Asp Ile | PND-SC | 3 |
| c) | r-Asn Asn Thr Arg Lys Ser Ile Arg Ile Gln Arg Gly Pro Gly Arg Ala Phe Val Thr Ile Gly Lys Ile Gly Asn | PND135 | 4 |
| d) | r-Arg Ile Gln Arg Gly Pro Gly Arg Ala Phe Val Thr Ile Gly Lys Ile Gly Asn | PND135-18 | 5 |
| e) | r-Arg Ile Gln Arg Gly Pro Gly Arg Phe Val Thr | PND135-12 | 6 |
| f) | r-His Ile Gly Pro Gly Arg Ala Phe | PND-MN8 | 7 |
| g) | r-Gly Pro Gly Arg Ala Phe | PND-MN6 | 8 |
| h) | r-Ile Gln Arg Gly Pro Gly Arg Ala Phe | PND-LAV-1 | 9 |

i) r-Ile Tyr Ile Gly Pro Gly Arg Ala Phe PND-SF2 10

j) r-Ile Ala Ile Gly Pro Gly Arg Thr Leu PND-NY5 11

k) r-Val Thr Leu Gly Pro Gly Arg Val Trp PND-CDC4 12

l) r-Ile Thr Lys Gly Pro Gly Arg Val Ile PND-RF 13

m) r-Thr Pro Ile Gly Leu Gly Gln Ser Leu PND-ELI 14

n) r-Thr Pro Ile Gly Leu Gly Gln Ala Leu PND-Z6 15

o) r-Ile His Phe Gly Pro Gly Gln Ala Leu PND-MAL 16

p) r-Ile Arg Ile Gly Pro Gly Lys Val Phe PND-Z3 17

q) 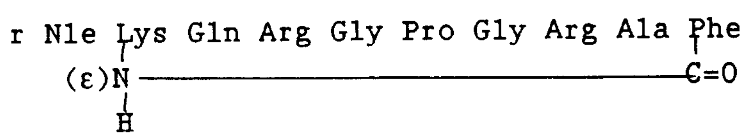 cPND15 23

r) 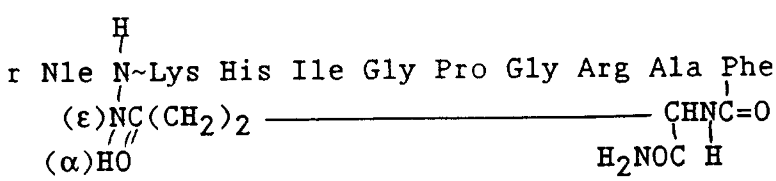 cPND31 or

s) 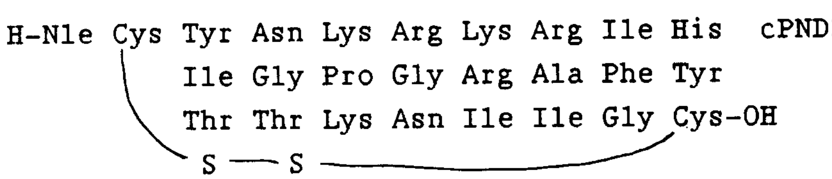 cPND33 22

wherein r is the position of linkage between the peptide and the MIEP, except that in option (s), the

linkage to MIEP may be throught the amino-terminal Nle, or any of the internal Lys residues of that peptide.

**10.** A conjugate having the structure:

a) 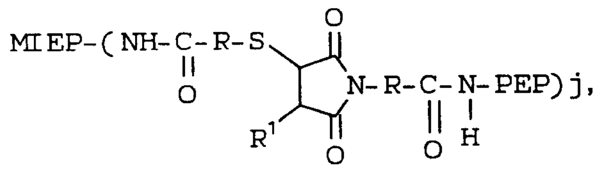

or

b) 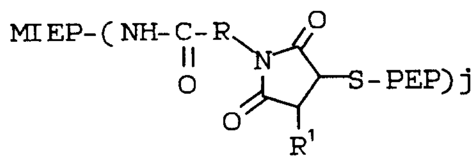

,

or a pharmaceutically acceptable salt thereof, wherein:

PEP consists essentially of an HIV PND;

j is the peptide loading and is between 1% and 50% of the total conjugate protein;

-R- is:

a) -lower alkylene-,
b) -substutited lower alkylene-,
c) -cycloalkylene-,
d) -substituted cyloalkylene-,
e) -phenylene-;

$-R^1$ is:

a) -hydrogen,
b) -lower alkyl, or
c) $-SO_3H$; and

-S- is sulfur; and

MIEP is the Class II protein of OMPC, the outer membrane protein complex of Neisseria meningitidis b.

**11.** The conjugate of Claim 10 having the structure:

a)

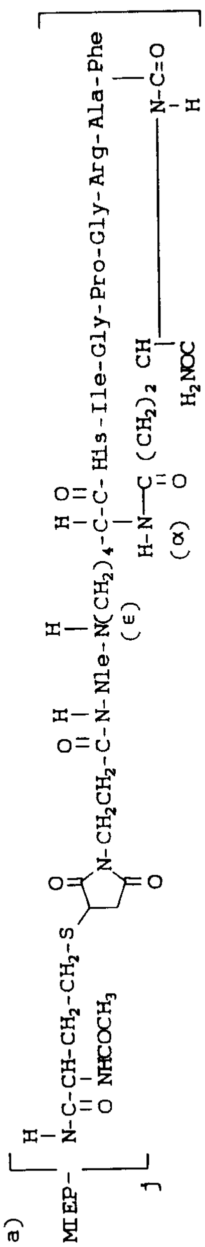

b)

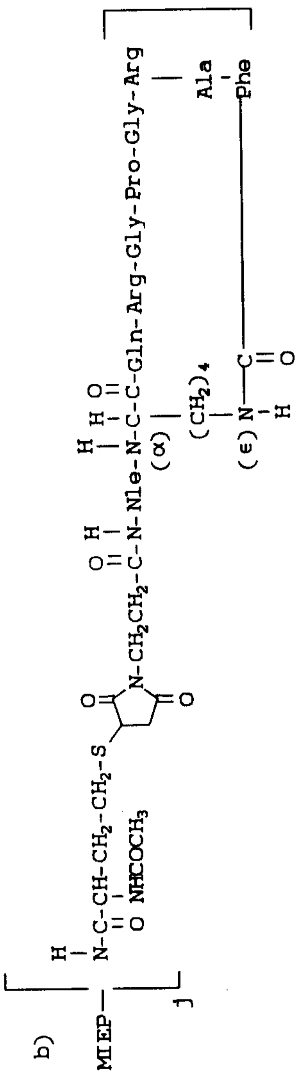

c)

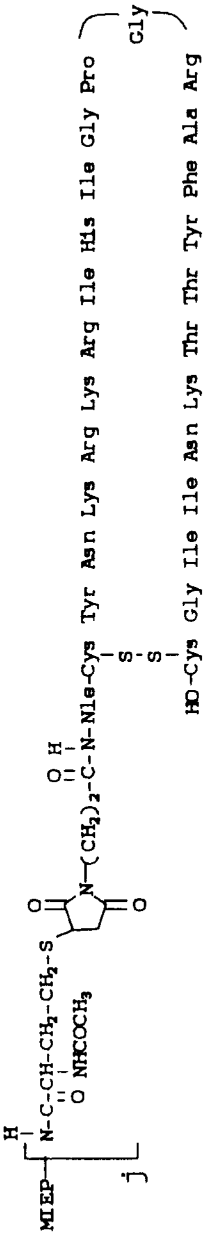

d)

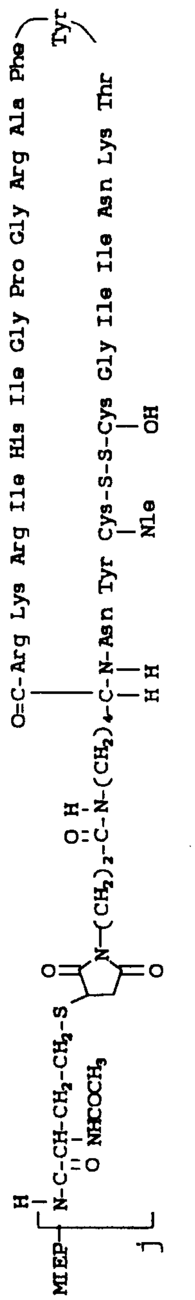

e)

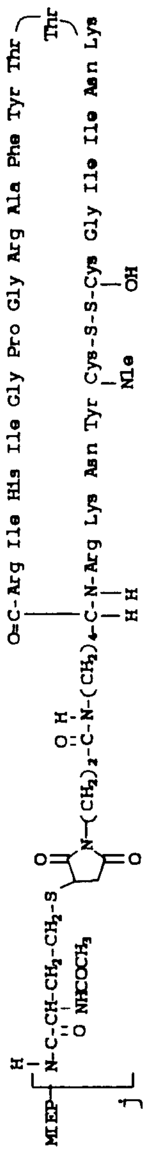

or

f)

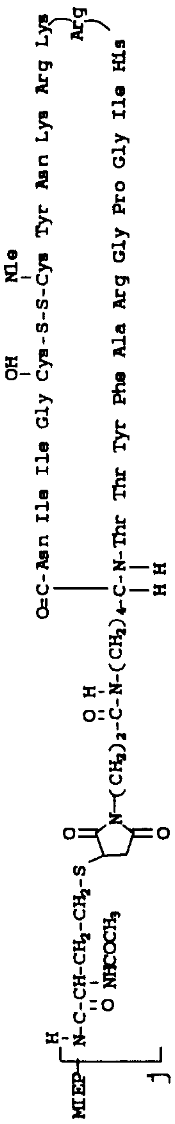

or a pharmaceutically acceptable salt thereof, wherein:

all variables are as defined in Claim 10.

**12.** A process for making a conjugate having the structure:

j(PEP-A-)MIEP

or pharmaceutically acceptable salts thereof, wherein:

PEP is an HIV PND peptide, or a peptide capable of raising mammalian immune responses which recognize HIV PNDs;

-A- is a convalent linkage, preferably a bigeneric spacer;

j is the peptide loading, and is the percentage by mass of peptide in the conjugate, and is

between 1% and 90% of the total protein mass in the conjugate;

which comprises the steps of:

1a. reacting the MIEP nucleophilic groups with a reagent which appends sulfhydryl groups on the MIEP, and

1b. reacting the product of step 1a with peptides previously derivatized so as to append an electrophilic group on the peptide; or

2a. reacting the MIEP nucleophilic groups with a bifunctional electrophilic reagent so as to generate an electrophilic MIEP; and

2b. reacting the product of step 2a. with a peptide containing a nucleophile.

**13.** The process of Claim 12 for making a conjugate wherein the MIEP is the Neisseria meningitidis b OMPC class II protein to generate a conjugate having the structure:

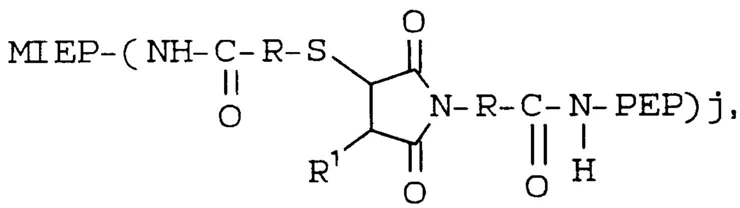

or pharmaceutically acceptable salts thereof.

PEP consists essentially of an HIV PND;

j is the peptide loading and is between 1% and 50% of the total conjugate protein;

-R- is:

a) -lower alkylene-,
b) -substutited lower alkylene-,
c) -cycloalkylene-,
d) -substituted cyloalkylene-,
e) -phenylene-;

$-R^1$ is:

a) -hydrogen,
b) -lower alkyl, or
c) $-SO_3H$; and

-S- is sulfur;

which comprises the steps of:

1a. reacting the MIEP nucleophilic groups with N-acetyl homocysteine thiolactone, which generates thiol groups on the MIEP, and

1b. reacting the product of step 1a. with peptides previously derivatized so as to append an electrophilic group on the peptide.

European Patent Office

# EUROPEAN SEARCH REPORT

Application Number

EP 92201693.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EP - A - 0 471 407 (MERCK & CO. INC.) * Claims 1-5 * -- | 1-13 | C 07 K 17/06<br>C 07 K 3/28<br>A 61 K 39/385<br>A 61 K 39/21 |
| P,X | EP - A - 0 467 714 (MERCK & CO. INC.) * Claims 1-7 * -- | 1-2 | |
| P,X | EP - A - 0 471 453 (MERCK & CO. INC.) * Claims 1-10 * -- | 3-9 | |
| A | EP - A - 0 372 501 (BEHRINGWERKE) * Claims 1-3 * -- | 1-13 | |
| P,A | CHEMICAL ABSTRACTS, vol. 115, no. 25, December 23, 1991 Columbus, Ohio, USA JOHN J. DONNELLY et al. "Adjuvant activity of the outer membrane protein complex of Neisseria meningitidis serogroup B for a polysaccarideprotein conjugate.", page 759, column 1, abstract-no. 277 347f & Vaccines 91: Mod. Approaches New Vaccines Incl. Prev. AIDS, 8th 1990, (Pub. 1991), 403-8 ---- | 1-13 | **TECHNICAL FIELDS SEARCHED (Int. Cl.5)**<br>C 07 K 3/00<br>C 07 K 7/00<br>C 07 K 9/00<br>C 07 K 17/00<br>A 61 K 37/00<br>A 61 K 39/00<br>C 12 N 7/00<br>C 12 N 15/00<br>C 12 P 21/00 |

The present search report has been drawn up for all claims

| Place of search | Date of completion of the search | Examiner |
|---|---|---|
| VIENNA | 25-09-1992 | SCHARF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document